# EUROPEAN PATENT SPECIFICATION

(11) **EP 1 295 611 B1**
(45) Date of publication and mention of the grant of the patent: **25.08.2010**
(21) Application number: 01938732.3
(22) Date of filing: 19.06.2001
(51) Int. Cl.: A61K 48/00, A61K 31/711, A61K 31/7105, A61K 47/42

(54) **OLIGONUCLEOTIDE-TRANSFERRING PREPARATIONS**
VERBINDUNGEN FÜR DEN TRANSFER VON OLIGONUKLEOTIDEN
PREPARATIONS DESTINEES AU TRANSFERT D'OLIGONUCLEOTIDES

(30) Priority: 20.06.2000 JP 2000184502
(43) Date of publication of application: 26.03.2003
(73) Proprietor: Dainippon Sumitomo Pharma Co., Ltd., Osaka-fu (JP); KOKEN CO., LTD., Toshima-ku, Tokyo 171-0031 (JP); National Cancer Center, Chuo-ku Tokyo 104-0045 (JP)
(72) Inventor: KUBOTA, Shunichiro, Hino-shi, Tokyo 191-0033 (JP); TERADA, Masaaki, Nerima-ku, Tokyo 177-0053 (JP); OCHIYA, Takahiro, 218, National Cancer Center, Chuo-ku, Tokyo 104-0045 (JP); ITOH, Hiroshi, Toshima-ku, Tokyo 171-0032 (JP); FURUSE, Masayasu, Sagamihara-shi, Kanagawa 228-0814 (JP); SANO, Akihiko, Toyonaka-shi, Osaka 560-0011 (JP); NAGAHARA, Shunji, 3-348, Sumitomokagaku,, Ibaraki-shi, Osaka 567-0841 (JP)
(74) Representative: Vossius & Partner
(86) International application number: PCT/JP2001/005195
(87) International publication number: WO 2001/097857

(56) References cited:
- EP-A2- 0 412 554
- WO-A1-97/02047
- WO-A1-97/15330
- WO-A2-95/22611
- OCHIYA T ET AL: "Antisense approaches to in vitro organ culture." METHODS IN ENZYMOLOGY, vol. 314, November 1999 (1999-11), pages 401-411, XP008106781 ISSN: 0076-6879
- JULIANO R L ET AL: "ASPECTS OF THE TRANSPORT AND DELIVERY OF ANTISENSE OLIGONUCLEOTIDES" CURRENT OPINION IN MOLECULAR THERAPEUTICS, CURRENT DRUGS, LONDON, GB, vol. 2, no. 3, 9 June 2000 (2000-06-09), pages 297-303, XP009016491 ISSN: 1464-8431

## Description

### FIELD OF THE INVENTION

The present invention relates to preparations for transferring oligonucleotides into a target cell which comprises a collagen as an essential component, said preparations being used in an antisense therapy. More specifically, the present invention relates to a safe preparation comprising a collagen as an essential component, whereby oligonucleotides can be efficiently transferred into a target cell.

### BACKGROUND ART

With rapid progress in the recent technique for analysis of genetic information, information of gene that causes disease has been accumulated. For example, information on genes essential for growth or survival of bacteria and viruses has been clarified in the field of infectious diseases. Moreover, in various diseases, information on disease-related genes, and on their over-expression and mutations has been clarified, and the relation between such genetic information and the mechanism of pathogenesis has been investigated.

Antisense therapy is to control or inhibit the expression of the disease-related gene information as shown above to as to treat or prevent the diseases.

Specifically, it comprises:
(1) administering an about 10 to 30 mer oligonucleotide comprising a base sequence complementary to a m-RNA or a m-RNA precursor of genes, of which expression is to be controlled or inhibited;
(2) allowing the oligonucleotide to form double strand together with the m-RNA or m-RNA precursor in cells; and
(3) inhibiting the translation of m-RNA by ribosome, or inhibiting the cleavage of double strand by RNase H, or the splicing of m-RNA precursor; whereby suppressing gene expressions.

In addition, there exists a method which comprises directly linking to the double-stranded DNA of the target gene to form a triple strand, whereby inhibiting the transcription into m-RNA.

Antisense therapy has been intensively studied since it had been proposed in the latter 1970s by Ts'O and Miller et al., Biochemistry, 16, 1988-1996, 1977; and Zamecnik et al., Proc. Natl. Acad. Sci. USA, 75, 280-284, 1978. Originally, studies on antisense therapy were performed using oligonucleotides of native DNA or RNA. However, they have some problems such as:
(1) rapid degradation and inactivation of native oligonucleotides due to endogenous nucleases;
(2) difficulty for negatively charged oligonucleotides to penetrate through cell membrane into cells due to negatively charged membrane; and the like.

Considering such problems, variously modified oligonucleotides having the basic structure of DNA or RNA have been developed and utilized. Among these modified oligonucleotides, phosphorothioate-type oligonucleotides are the most practical ones, wherein diester bonds between respective nucleosides are substituted with phosphorothioate bonds (hereinafter referred to as S-oligonucleotide). S-oligonucleotides have been utilized in most of clinical trials that were conducted until now.

However, even S-oligonucleotides have the following problems:
(1) that they are degraded and inactivated by endogenous nuclease, although not so rapidly as native oligonucleotides;
(2) that they have a less ability to form a double strand together with m-RNA compared to native oligonucleotides;
(3) that they should be administered to the target cells at higher concentration because of their low ability to form a double strand;
(4) that they cause release of cytokines, inhibition of blood coagulations, activation of complements, and allergic reaction or the like when administered systemically at higher concentration;
(5) that they specifically or non-specifically bind endogenous proteins, and consequently could not form a double strand with the target m-RNA but produce non-specific effects.

They fail to completely satisfy the requirements of antisense therapy, thus they have not been practically utilized. These problems are true not only for oligonucleotides used as the first generation of S-oligonucleotides, but so-called the second and the third generation for antisense therapy, which have been developed until now (Kazunari Yokoyama, Kagaku-to-Seibutsu, 36, 556-559, 1998). Because of the problems, it has been generally recognized that suppression of target gene expression using oligonucleotides would not provide good results *in vivo*, particularly in clinical trials, even though it could provide good results *in vitro.* Actually, clinical trials for antisense drugs often suspended because they fail to show good results.

Murakami reported that the suspension of clinical trials would be caused by the problem in delivery of antisense drugs, and the successful development of antisense drugs depends on the development of delivery methods (Akira Murakami, Seitai-no-Kagaku, 49, 309-314, 1998). The methods to deliver oligonucleotides have been intensively investigated mainly aiming at improvement in penetration and transportation of oligonucleotides into cells. To solve such problems, the following methods were proposed in Yokoyama's review (Kazunari Yokoyama, Cellular Engineering, 16, 1463-1473, 1997):
(1) to conjugate oligonucleotides with poly-charged compounds such as poly-L-lysine and polyethyleneimine;
(2) to use transferrin/poly-L-lysine-conjugated DNAs in the presence of capsid of replication-defective adenovirus;
(3) to use fragments of the homeo domains of membrane-fused peptide derived from influenza virus HA surface protein (Bongartz, J.P. et al., Nucleic Acids Research, 22, 4681-4688, 1994) and *Antennapedia* protein of *Drosophila;*
(4) to bind oligonucleotides with folic acid or acialoglycoprotein receptor, or transferrin, so as to be targeted to specific cell surface receptors;
(5) to conjugate oligonucleotides with cholesterol;
(6) to encapsulate oligonucleotides into cationic lipids, and the like. These proposals mainly aim at improving penetration and transportation of oligonucleotide into cells, and *in vitro* experiments wherein oligonucleotides can be directly administered to cells, have provided good results. However, *in vivo* experiments have failed to show sufficient results, and have been hard to be practically utilized.

For example, the methods comprising administering oligonucleotides encapsulated into cationic lipids (i.e., liposomes) have been intensively studied since they efficiently transfer oligonucleotides into cells. However, those methods have problems, for example, that oligonucleotides fail to exert on target cells at high concentration because they diffuse throughout the body immediately after administration, that proteins existing in the body bind to the liposome to inhibit adhesion to target cells, and that cationic lipids constituting the liposome may have cytotoxicity, and thus they have failed to provide practically successful results. Actually, clinical trials using S-oligonucleotides have never utilized any liposome (Seitai-no-kagaku, 49, 309-314, 1998). There still exist needs for development of practical methods for delivering oligonucleotides.

On the other hand, a method for transferring a gene wherein a plasmid DNA or an viral vector is gradually released from biocompatible or bone-compatible materials has been described in Japanese Patent Publication (kokai) No. 71542/1997, and USP No. 5,763,416. These documents describe that biocompatible or bone-compatible materials gradually release plasmid DNAs having a molecular weight from 2,650,000 to 5,300,000 (corresponding to 4,000 bp - 8,000 bp) or a viral vectors with the larger molecular size to exhibit good expression efficiencies. However, the number of plasmid DNAs or viral vectors to be transferred into one target cell, to which these plasmid DNAs or viral vectors are required to be transferred, is considerably smaller compared to the number of oligonucleotides required to be transferred in antisense therapy. It is sufficient to introduce one plasmid DNA or viral vector into a single target cell, because information on genes encoded in the plasmid DNA or viral vector is transcribed to many m-RNAs and expressed after they are introduced into the target cell. On the other hand, in order to transfer oligonucleotides into cells and form rigid double strands to suppress the gene expression in antisense therapy, oligonucleotide concentration in cells should be raised as high as possible. The Japanese Patent Publication (kokai) 71542/1997 and USP No. 5,763,416 as shown above suggest specifically neither that biocompatible or bone-compatible materials gradually release oligonucleotides extremely smaller (single stranded, having a molecular weight of about 3,300 to about 9,900) compared to the plasmid DNAs or viral vectors could inhibit the gene expression, nor that oligonucleotide concentration in the target cell could be raised to a clinically sufficient level.

Moreover, the above publications do not suggest any preparation to transfer oligonucleotides, which can be used in antisense therapy, and have the properties as described below:
1) to protect oligonucleotides administered to living bodies from degradation with nucleases;
2) to protect oligonucleotide administered to living bodies from specific or non-specific binding with endogenous proteins;
3) to maintain a high concentration of oligonucleotides around the target cells in order to increase an oligonucleotide concentration in the cells so that the expression of a target m-RNA is inhibited by forming a rigid double strand;
4) to restrict such a high concentration of oligonucleotide only to the cells around the target cells in order not to produce systemic side effects; and
5) to be required to extend the period of time when the m-RNA expression could be suppressed upon a single dosage.

### DISCLOSURE OF THE INVENTION

The object of the present invention is to provide a preparation for transferring efficiently an oligonucleotide necessary in antisense therapy into cells, thus contributing to the treatment of various diseases.

The inventors of the present application studied intensively to solve the above problems. As the results, we have found that a preparation as defined in claim 1, which comprises an oligonucleotide having a base a sequence complementary to a sequence of a target m-RNA and a collagen (as well as a pharmaceutically acceptable additive) efficiently inhibits the expression of the target m-RNA without inducing any side effect *in vivo*, and maintains its effect for a long period of time. The inventors have conducted further study and accomplished the present invention.

### BRIEF DESCRIPTION OF DRAWINGS

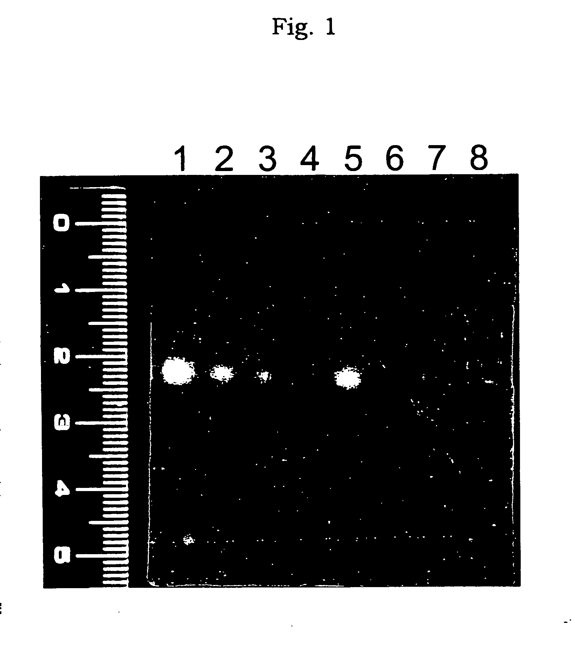

Figure 1 shows a 3% agarose-gel electrophoresis in Experiment 1. Fig. 1 shows the effect of nuclease on phosphorothioate antisense oligonucleotide in oligonucleotide preparations of Example 1 and Reference Example 1. Lane 1: Example 1 - after 0 min., Lane 2: Example 1 - after 30 min., Lane 3: Example 1 - after 60 min., Lane 4: Example 1 - after 180 min., Lane 5: Reference Example 1 - after 0 min., Lane 6: Reference Example 1 - after 30 min., Lane 7: Reference Example 1 - after 60 min., Lane 8: Reference Example - after 180 min.

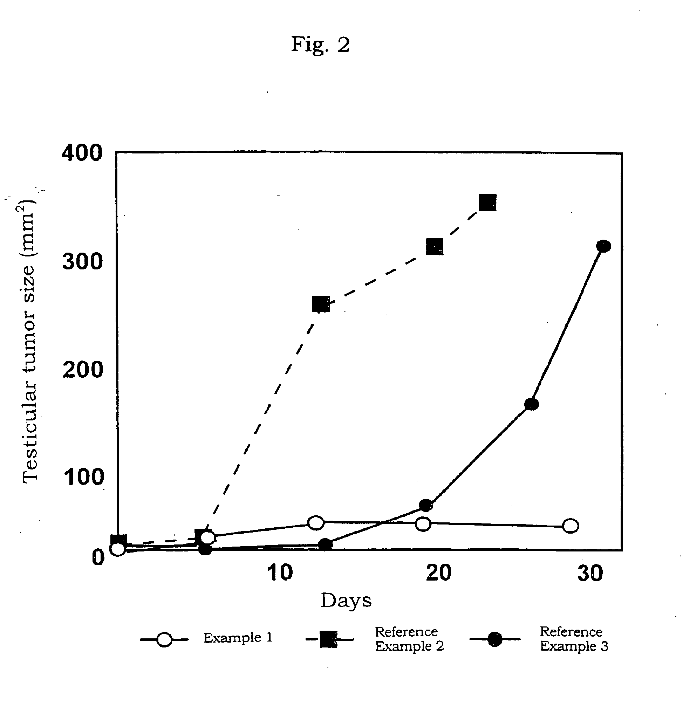

Figure 2 is a graph showing that administration of the preparation for transferring an oligonucleotide of Example 1 to nude mouse testis, to which human testicular tumor cells (NEC-8) had been transplanted, inhibited the growth of NEC-8 cells for a long time.

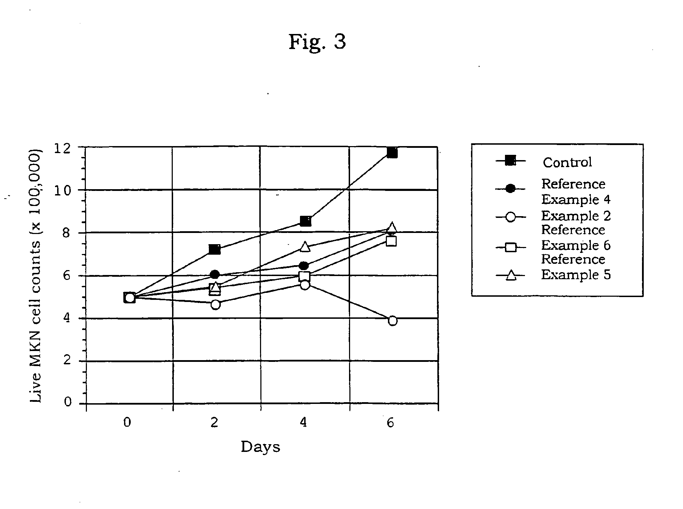

Figure 3 is a graph showing the *in vitro* addition of the preparation for transferring an oligonucleotide of Example 2 to the human stomach cancer cells inhibited the growth of human stomach cancer cells, and decreased the number of cells compared to the atelocollagen solution of Reference Example 4, the antisense oligonucleotide solution of Reference Example 5, the scramble oligonucleotide composition of Reference Example 6.

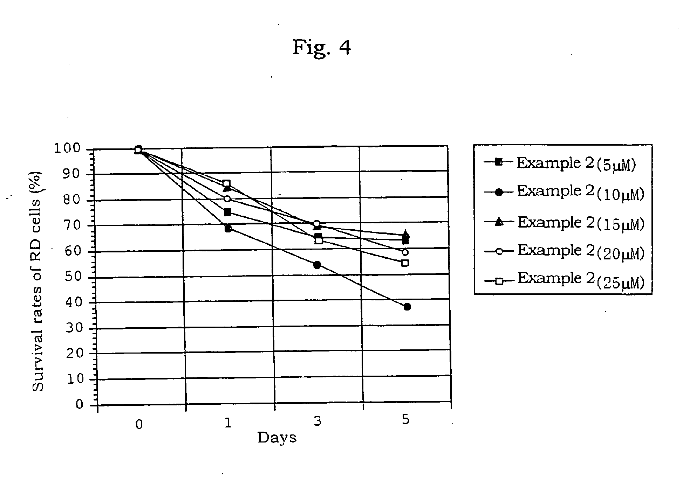

Figure 4 is a graph showing the comparison of the *in vitro* inhibition of growth of human rhabdomyosarcoma cells between the concentrations of the phosphorothioate antisense oligonucleotide contained in the preparations for transferring an oligonucleotide of Example 2.

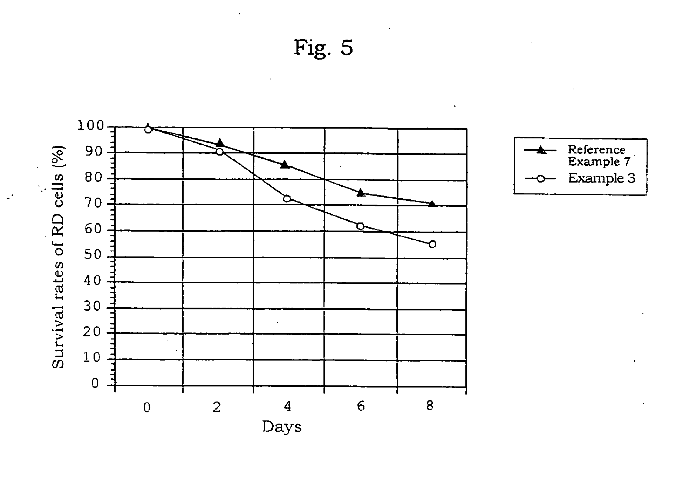

Figure 5 is a graph showing that the preparation for transferring an oligonucleotide containing a liposome according to Example 3, when added to human rhabdomyosarcoma cells *in vitro,* exhibited the stronger cytotoxic effect, compared to the liposome preparation of Reference Example 7.

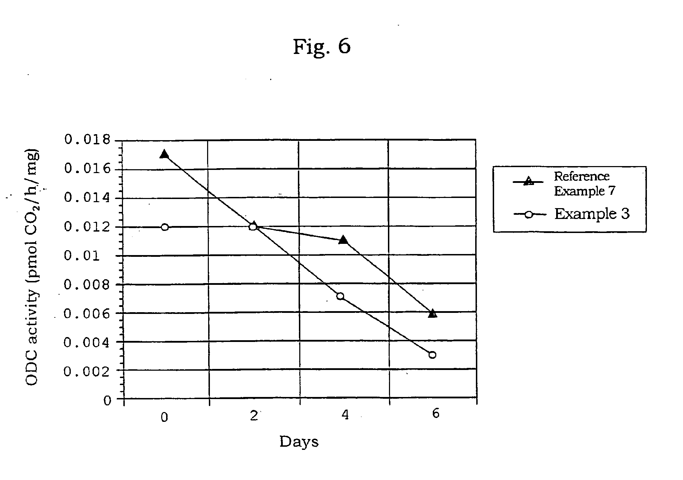

Figure 6 is a graph showing that the preparation for transferring an oligonucleotide containing a liposome according to Example 3, when added to human rhabdomyosarcoma cells *in vitro,* reduced the activity of ornithine decarboxylase more strongly than the liposome preparation of Reference Example 7.

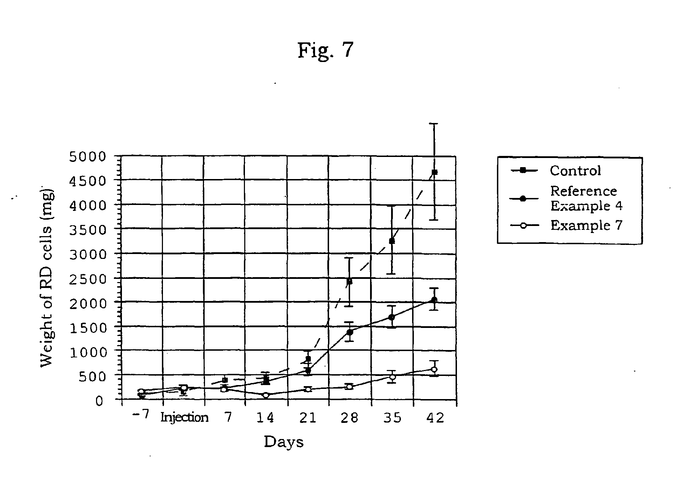

Figure 7 is a graph showing that the preparation for transferring an oligonucleotide of Example 7, when administered to the nude mice that had been transplanted with human rhabdomyosarcoma cells at the site of transplantation, inhibited the growth of rhabdomyosarcoma cells for a longer period of time than the atelocollagen solution of Reference Example 4.

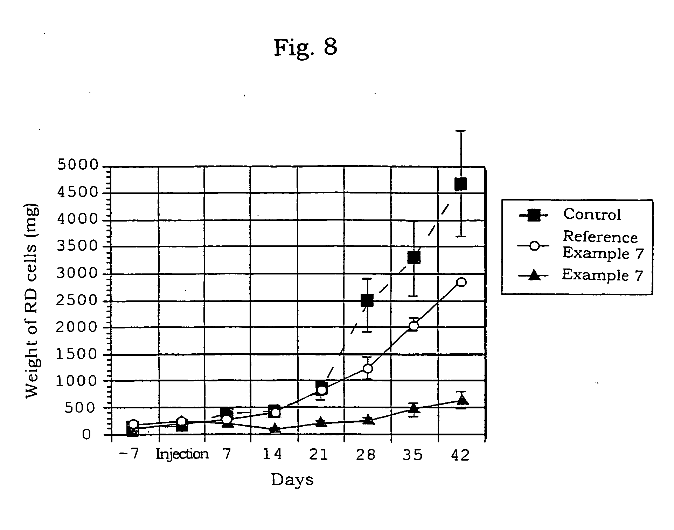

Figure 8 is a graph showing that the preparation for transferring an oligonucleotide of Example 7, when administered to the nude mice that had been transplanted with human rhabdomyosarcoma cells at the site of transplantation, inhibited for a longer period of time than the liposome preparation of Reference Example 7.

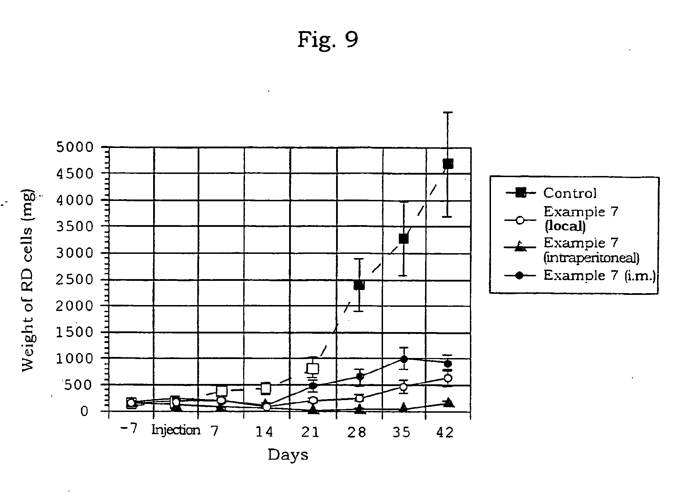

Figure 9 is a graph showing that the preparation for transferring an oligonucleotide of Example 7, when administered to the nude mice that had been transplanted with human rhabdomyosarcoma cells in the site of the transplantation, the back muscle opposed to the transplantation site and in peritoneal cavity, inhibited the growth of rhabdomyosarcoma cells at all of the sites for a longer period of time compared to the liposome preparations administered at rhabdomyosarcoma-transplanted site.

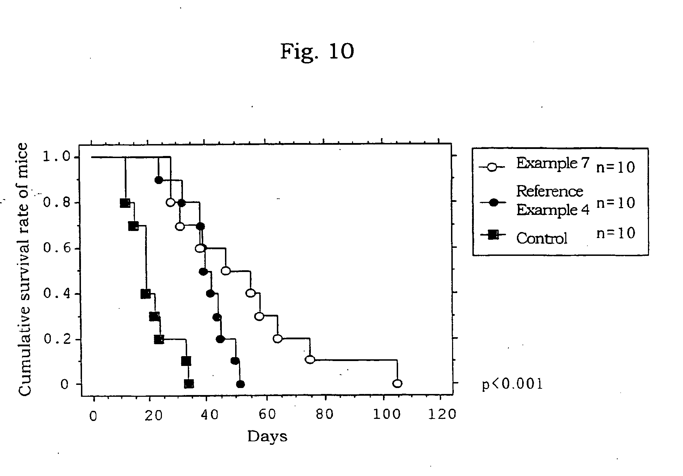

Figure 10 is a graph showing that the preparation for transferring an oligonucleotide of Example 7, when administered to the nude mice that had been transplanted with human rhabdomyosarcoma cells at the site of the transplantation, enhanced survival of the nude mice compared to the atelocollagen solution of Reference Example 4.

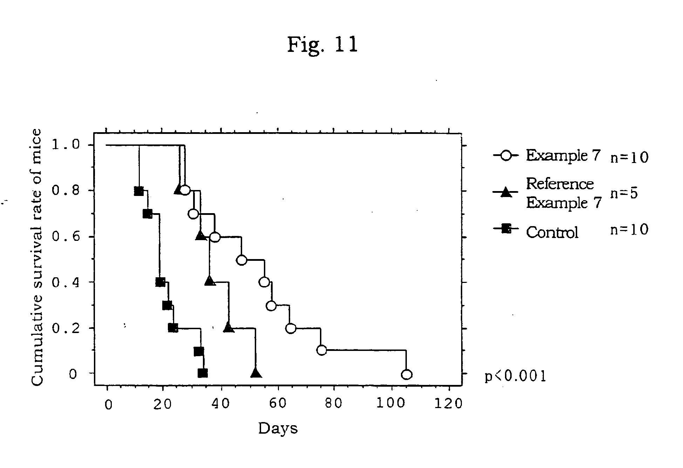

Figure 11 is a graph showing that the preparation for transferring an oligonucleotide of Example 7, when administered to the nude mice that had been transplanted with human rhabdomyosarcoma cells at the site of transplantation, enhanced survival of the nude mice compared to the liposome preparation of Reference Example 7.

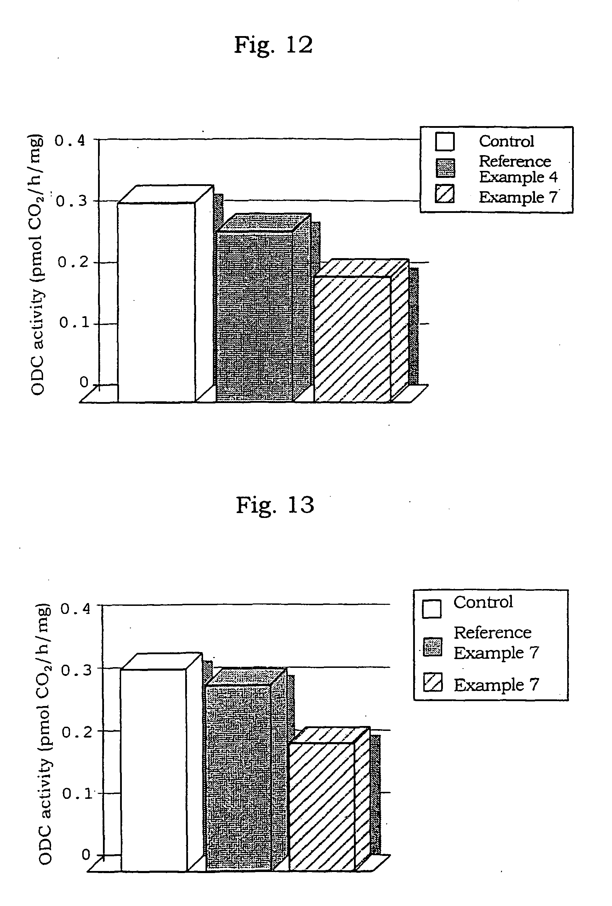

Figure 12 is a graph showing that the preparation for transferring an oligonucleotide of Example 7, when administered to the nude mice that had been transplanted with human rhabdomyosarcoma cells at the site of transplantation, inhibited the production of ornithine decarboxylase in the rhabdomyosarcoma cells more strongly compared to the atelocollagen solution of Reference Example 4.
Figure 13 is a graph showing that the preparation for transferring an oligonucleotide of Example 7, when administered to the nude mice that had been transplanted with human rhabdomyosarcoma cells at the site of transplantation, inhibited production of ornithine decarboxylase in the rhabdomyosarcoma cells more strongly compared to the liposome preparation of Reference Example 7.

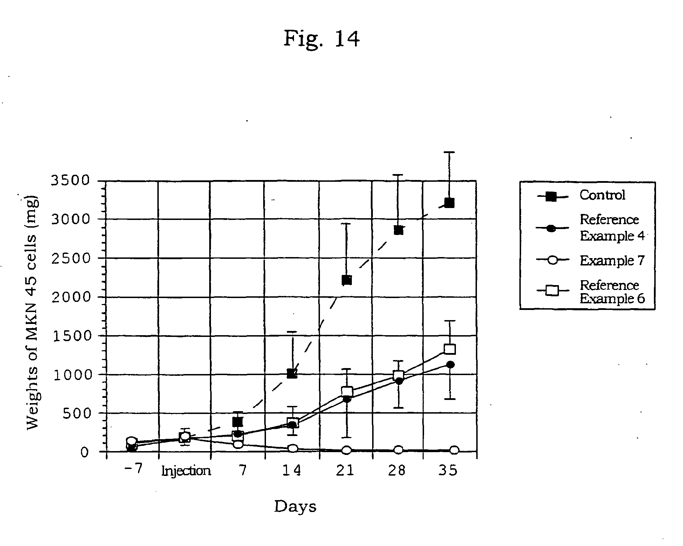

Figure 14 is a graph showing that the preparation for transferring an oligonucleotide of Example 7, when administered to the nude mice that had been transplanted with human stomach cancer cells at the site of transplantation, inhibited growth of the stomach cancer cells for a longer period of time compared to the atelocollagen solution of Reference Example 4 and the solution composition of Reference Example 6 containing a phosphorothioate oligonucleotide having a sequence heterologous to that of the gene of ornithine decarboxylase.

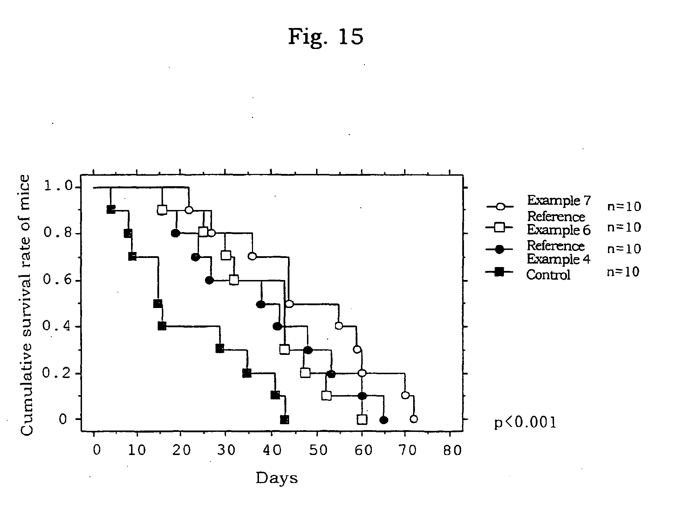

Figure 15 is a graph showing that the preparation for transferring an oligonucleotide of Example 7, when administered to the nude mice that had been transplanted with human stomach cancer cells at the site of transplantation, enhanced survival of the nude mice compared to the atelocollagen solution of Reference Example 4 and the solution composition of Reference Example 6 containing a phosphorothioate oligonucleotide having a sequence heterologous to that of the gene of ornithine decarboxylase.

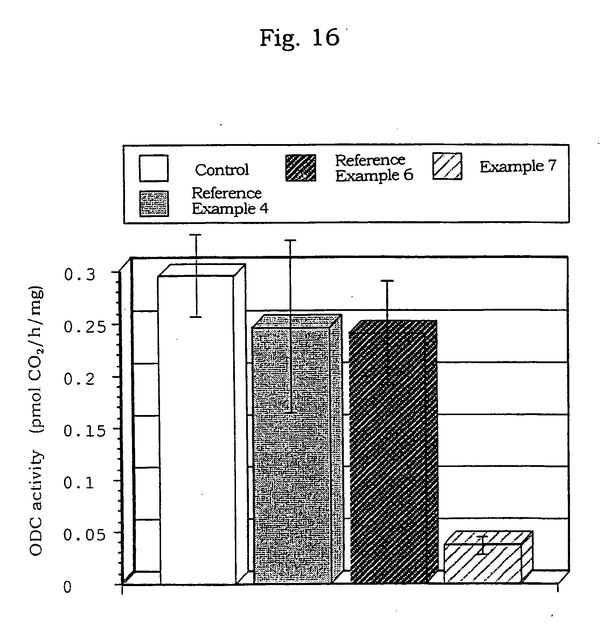

Figure 16 is a graph showing that the preparation for transferring an oligonucleotide of Example 7, when administered to the nude mice that had been transplanted with human stomach cancer cells at the site of transplantation, inhibited the production of ornithine decarboxylase in the stomach cancer cells more strongly compared to the atelocollagen solution of Reference Example 4 and the solution composition of Reference Example 6 containing a phosphorothioate oligonucleotide having a sequence heterologous to that of the gene of ornithine decarboxylase.

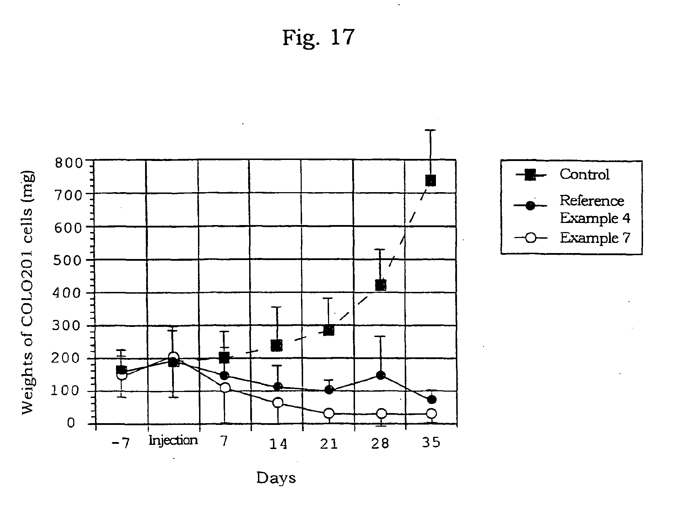

Figure 17 is a graph showing that the preparation for transferring an oligonucleotide of Example 7, when administered to the nude mice that had been transplanted with human large intestinal cancer cells at the site of transplantation, inhibited growth of the large intestinal cancer cells for a longer period of time compared to the atelocollagen solution of Reference Example 4.

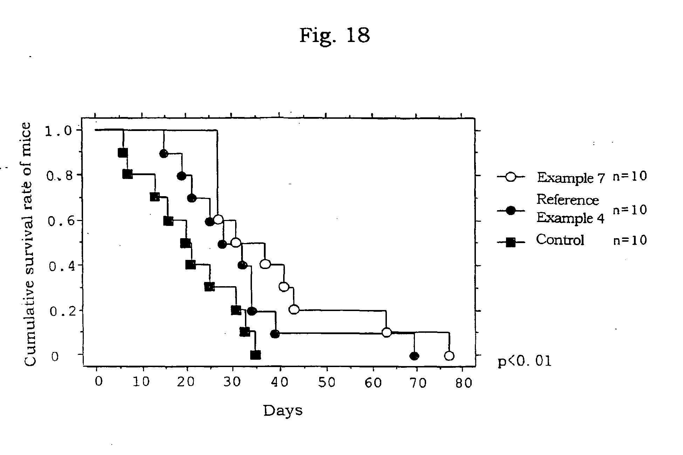

Figure 18 is a graph showing that the preparation for transferring an oligonucleotide of Example 7, when administered to the nude mice that had been transplanted with human large intestinal cancer cells at the site of transplantation, enhanced survival of the nude mice compared to the atelocollagen solution of Reference Example 4.

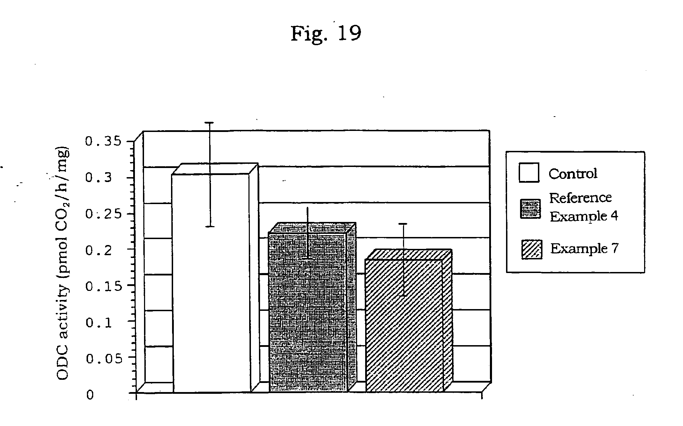

Figure 19 is a graph showing that the preparation for transferring an oligonucleotide of Example 7, when administered to the nude mice that had been transplanted with human large intestinal cancer cells at the site of transplantation, inhibited the production of ornithine decarboxylase in the large intestinal cancer cells more strongly than the atelocollagen solution of Control.

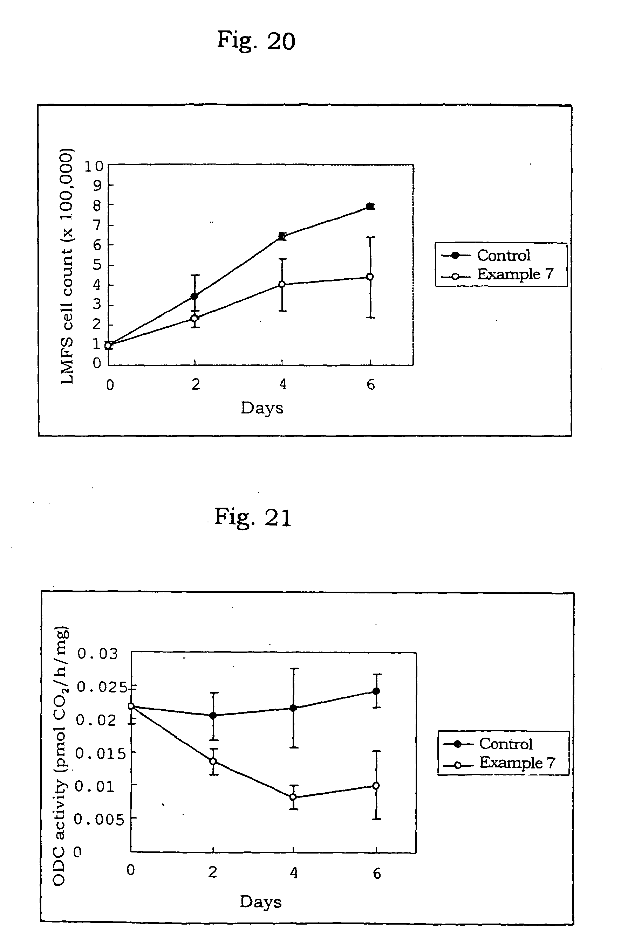

Figure 20 is a graph showing that the preparation for transferring an oligonucleotide of Example 7, when added to the mouse leiomyosarcoma cells (LMFS cells) *in vitro,* inhibited the growth of the leiomyosarcoma cells more strongly than Reference Examples.
Figure 21 is a graph showing that the preparation for transferring an oligonucleotide of Example 7, when added to the mouse leiomyosarcoma cells (LMFS cells) *in vitro,* inhibited the production of ornithine decarboxylase in the leiomyosarcoma cells compared to Reference Examples.

### BEST MODE FOR CARRYING OUT THE INVENTION

Accordingly, the present invention relates to:
(1) A preparation for transferring a desired oligonucleotide into a target cell as defined in claim 1.
(2) The preparation according to (1) wherein the target cell is an animal cell;
(3) The preparation according to (2) wherein the target cell is an organ or a tissue which require to be treated, or its cell around;
(4) The preparation according to any one of (1) to (3) wherein the collagen is atelocollagen:
(5) The preparation according to any one of (1) to (4) wherein the molecular weight of the collagen is from about 300,000 to about 900,000;
(6) The preparation according to any one of (1) to (5) wherein the preparation is a sustained-release formulation;
(7) The preparation according to any one of (1) to (6) wherein the oligonucleotide is from 5 to 30 mer in length;
(8) The preparation according to (6) or (7) wherein the oligonucleotide is a DNA or a DNA derivative;
(9) The preparation according to (6) or (7) wherein the oligonucleotide is an RNA or an RNA derivative;
(10) The preparation according to (8) or (9) wherein the DNA derivative or the RNA derivative has at least one phosphorothioate bond;
(11) The preparation according to any one of (1) to (10), wherein the oligonucleotide contains as at least portion a base sequence that binds complementarily under physiological conditions to a sense or antisense strand of a gene encoding a protein that exhibits a physiological effect to disrupt the homeostasis of a living body;
(12) The preparation according to any one of (1) to (10), wherein the oligonucleotide contains as at least portion a base sequence that binds complementarily under physiological conditions to a sense or antisense strand of a gene that is specific to a pathogenic virus or a pathogenic bacteria;
(13) The preparation according to any one of (1) to (10), wherein the oligonucleotide contains a sequence complementary to a region containing an initiation codon of a messenger RNA or to a splicing site of a messenger RNA precursor;
(14) The preparation according to (11) wherein the gene encoding a protein that exhibits a physiological effect to disrupt the homeostasis is a cancer gene;
(15) The preparation according to (14), wherein the cancer gene is selected from a group consisting of growth factors, receptor-type tyrosine kinases, non-receptor-type tyrosine kinases, GTP-binding proteins, serine-threonine kinases, and transcription factors;
(16) The preparation according to (14) wherein the cancer gene is a gene encoding hst-1 or ornithine decarboxylase;
(17) The preparation according to any one of (1) to (12) wherein the oligonucleotide is contained in a material that promotes the transfer of an oligonucleotide into cells or the transportation to nucleus, or wherein the oligonucleotide is complexed with the material;
(18) The preparation according to (17) wherein the material that promotes the transfer into cells is a cationic liposome, a membrane-fused liposome or a cationic polymer;
(19) The preparation according to (1) wherein the collagen is a type 1 collagen derived from dermis, or a type 1 collagen prepared by genetic engineering;
(20) The preparation according to (1) further comprising a pharmaceutically acceptable additive;
(21) The preparation according to (1) wherein the additive is a mono-, di-, tri-saccharide or a higher oligosaccharide or a corresponding sugar alcohol, a non-hydrophobic amino acid, or an organic acid having two or more carboxyl groups;
(22) The preparation according to (1) wherein the preparation is in the form of solution, suspension, sponge, pellet or powder;
(23) The preparation according to (22) wherein the preparation is a solution or a suspension having a pH of 4 to 8;
(24) The preparation according to (22) wherein the collagen content is in a range from 0.001 % to 10 % (w/w); and
(25) The preparation according to (22) wherein the preparation is in the form of sponge, pellet or powder, and has a collagen content of 5 to 99 % (w/w).

Further, the present invention relates to antisense therapy for treating a cancer, which comprises administering the preparation for transferring an oligonucleotide according to the present invention. More particularly, it relates to antisense therapy which comprises administering the preparation comprising a desired oligonucleotide and a collagen to a living body via transdermal, subcutaneous, intradermic, intramuscular, intraperitoneal, intracerebral, interstitial, intravascular, oral, rectal, or gastrointestinal route, whereby transferring efficiently said oligonucleotide into a cell.

A preparation for transferring an oligonucleotide, more particularly, a solution or a suspension preparation as obtained by mixing an oligonucleotide solution with a collagen solution (as well as a pharmaceutically acceptable additive) according to the present invention, is characterized by the following properties:
1) it can be formed into a film, a sponge, a powder, a Minipellet or the like, depending on the particular object;
2) after administered in the target tissue or its around, the preparation will remain at the site where administered;
3) it protects oligonucleotide from being degraded by a nuclease;
4) it gradually release oligonucleotide;
5) The release rate of an oligonucleotide from the preparation can be controlled depending on the particular object;
6) The concentration of an oligonucleotide around a target tissue can be maintained at a high level;
7) it efficiently inhibits the expression of the target m-RNA *in vivo* without inducing any side-effect and retains the inhibition for a long period of time.

Moreover, the present invention relates to a method for treatment and prevention of infectious diseases comprising using the preparation as described above; or a method for treatment and prevention of various diseases induced by over-expression of the certain gene information; and particularly to a method for treatment and prevention of a cancer.

The preparation for transferring an oligonucleotide of the present invention may be a solution or a suspension obtained by mixing an oligonucleotide solution with a collagen solution, or may be a film, a sponge, a powder, or a Minipellet formed from the solution or the suspension. Therefore, a soluble collagen or a solubilized collagen may be desirably utilized as a collagen as used in the present invention.

A soluble collagen include those that are soluble in an acidic or neutral water or a water containing a salt. A solubilized collagen include, for example, an enzymatically solubilized collagen which may be solubilized with an enzyme, an alkali-solubilized collagen which may be solubilized with an alkali, and the like.

Preferably, these collagens can penetrate through a membrane filter having a pore size of 1 micrometer. Solubility of collagen may vary depending on the crosslinking degree of the collagen. Higher is the crosslinking degree, more difficult the collagen is solubilized. Accordingly, the crosslinking degree of a collagen used in the present invention is, for example, not more than 3 (trimer), more preferably not more than 2 (dimer).

Preferable molecular weight of the collagen is, for example, from about 300,000 to 900,000, and more preferably from about 300,000 to about 600,000.

Collagens as used herein include those extracted from any animal species and genetic recombinants thereof. Preferable collagen is extracted from vertebrates or genetic recombinants thereof, more preferably collagens extracted from a mammal, a bird, a fish or genetic recombinant thereof, and more preferable collagen is extracted from a mammal or a bird having a high denaturation temperature, or genetic recombinants thereof. Any type of collagen may be used, and, because of the type existing in animal bodies, type I - V collagens or genetic recombinants thereof are preferable.

For example, such collagens include a type 1 collagen obtained by acid extraction from a mammal dermis or a genetic recombinant thereof. More preferably, they include, for example, a type 1 collagen obtained by acid extraction from calf dermis, a type 1 collagen produced by genetic engineering, and the like. As a type 1 collagen produced by genetic engineering, those derived from calf dermis or from human dermis are preferably. Collagens derived from tendon, which are also type 1 collagens, are not suitable herein, because they have a high degree of crosslinking and are insoluble.

Further, an atelocollagen that is obtained by removing enzymatically a telopeptide having high antigenicity or an atelocollagen produced by genetic engineering is preferable for the sake of safety. An atelocollagen having three or less tyrosine residues per molecule is more preferable.

The collagen may be obtained by culturing a primary culture cell line or an established cell line producing a collagen, followed by separating and purifying from the culture (the culture supernatant, the culture cells). Moreover, a gene encoding a collagen is incorporated into an appropriate vector by genetic engineering, and then the vector is transferred into proper hosts for transformation, whereby obtaining an intended recombinant collagen from the culture supernatant of the transformants. The host cell as used herein are not limited to particular ones, and include various host cell which have been conventionally utilized in genetic engineering, such as an E. coli, a Bacillus subtilis, a yeast, a plant or an animal cell.

An oligonucleotide used in the present invention is 5 mer to 30 mer in length, and more specifically 15 mer to 25 mer in length.

An oligonucleotide used in the present invention includes, for example, a DNA, a DNA derivative, an RNA, an RNA derivative and the like. More specifically, a DNA derivative or an RNA derivative is such as an oligonucleotide having at least one or more phosphorothioate bonds.

More specifically, an oligonucleotide used in the present invention may contain at least portion a base sequence that binds complementarily under physiological condition to a sense or antisense strand of a gene encoding a protein that has a physiological effect to disrupt the homeostasis of living bodies, a gene specific to pathogenic viruses, bacteria or the like, and, more specifically, it contains a sequence that binds complimentarily to the messenger RNA of a gene specific to a pathogenic virus, a bacterium or the like, or a gene encoding a protein having a physiological effect to disrupt the homeostasis of a living body.

More specifically, an oligonucleotide used in the present invention may contain a sequence complementary to a region containing an initiation codon of a messenger RNA, or to a splicing site of a precursor messenger RNA. Examples of the oligonucleotides used in the present invention include, for example, those used for treatment or prevention of a cancer, such as an oligonucleotide having a sequence of 5'-CTCGTAGGCGTTGTAGTTGT-3' (SEQ ID NO:1) which specifically inhibits the expression of hst-1; ISIS3521 which specifically inhibits the expression of protein kinase Cα to effectively treat progressive cancers such as non-small cell lung carcinoma and colon cancer, and has been applied in the trials to the treatment of prostate cancer, breast cancer, ovary cancer, pancreas cancer, large intestinal cancer, small cell lung carcinoma; ISIS5132/CGP69846A which specifically inhibits the expression of C-raf kinase and has been applied in the trials to the treatment of prostate cancer, breast cancer, ovary cancer, cephalophyma, pancreas cancer, large intestinal cancer, small cell lung carcinoma; ISIS 2503 which specifically inhibits the expression of Haras and has been applied in the trials to the treatment of large intestinal cancer, breast cancer, cephalophyma, pancreas cancer, and small cell cancer; GEM231 which specifically inhibits the expression of protein kinase A type I; MG98 which specifically inhibits the expression of DNA methyl transferase; INXC-6295 which inhibits the expression of c-myc; INX-3001 which inhibits the expression of c-myb and has been considered to be applied to the treatment of leukemia; G-3139 (Genasense) which inhibits expression of bc1-2 and has been considered to be applied to the treatment of non-Hodgkin's lymphoma, large intestinal cancer, small cell lung carcinoma, chronic lymphatic leukemia, acute myeloid leukemia, breast cancer, lymphoma, melanoma, myeloma, non-small cell lung carcinoma, prostate cancer; an oligonucleotide which inhibits the expression of MDM2 protein; an oligonucleotide which inhibits the expression of VEGF and the like. Further, examples of the oligonucleotides used for treatment or prevention of infectious diseases include GEM92 and GPI-2A which inhibits the growth of HIV. ISIS2922, Vitravene, ISIS13312 and GEM 132 (fomivirsen) which inhibits the growth of cytomegalovirus, ISIS14803 which inhibits the growth of hepatitis C virus, and the like. Examples of the oligonucleotides used for treatment of inflammation include ISIS2302 which specifically inhibits the expression of ICAM-1, and which has been applied in the trials to the treatment of Crohn's disease, ulcerative colitis, kidney transplantation rejection inhibition, psoriasis, asthma; EPI-2000 which inhibits the expression of adenosine A1 receptor and has been applied in the trials to the treatment of asthma; and oligonucleotides which inhibit the expression of TNF-α, CD49d (VLA-4), VCAM-1, PECAM-1 and the like. Further, examples of the oligonucleotides that prevent the restenosis after percutaneous transluminal coronary angiogenesis include Resten-NG that inhibits the expression of c-myc.

Genes encoding a protein that exhibits a physiological effect to disrupt the homeostasis include, for example, a series of genes, so-called cancer genes, and more specifically include genes for growth factors, receptor type tyrosine kinases, non-receptor type tyrosine kinases, GTP-binding proteins, serine-threonine kinases, transcription factors and the like. More specifically, genes coding for hst-1 or ornithine decarboxylase and the like are exemplified.

As used herein, the oligonucleotide may be generally contained in a material that promotes the transfer of oligonucleotide into cells or a material that promote the transportation of oligonucleotide into nucleus, or may form a complex together with said materials. Examples of the former include a cationic lipid, a cationic polymer, a hydrophobic polymer and the like. "Cationic lipids" include DOTMA (N-[1-(2,3-dioleyloxy)propyl]-N-trimethylammonium chloride), DOSPA (2,3-dioleyloxy-N-[2-(sperminecarboxamido)ethyl]-N,N-dimethyl-1-propanaminium trifluoroacetate), DDAB (dimethyldioctacresylammonium bromide), TM-TPS (N,NI,NII,NIII-tetramethyl-N,N',N",N"'-tetrapalmitylspermine), DMRIE (1,2-dimyristyloxypropyl-3-dimethylhydroxyethylammonium bromide), N-(α-trimethyl ammonium acetyl)-didodecyl-D-glutamate chloride) (Biochemical Biophysical Research Communication, 196, 1042 (1994)) and the like. Cationic liposomes consisting of the cationic lipids as described above and neutral lipids such as DOPE (dioleylphosphatidyl ethanol amine), or a mixture of cationic lipids and cholesterol may be used. "Cationic polymers" are polymers having electrostatic interaction with an oligonucleotides, and include, for example, lipopolyamines such as DOGS (dioctadecylamide glycylspermine) or the like; peptides such as AlkCWK18 or the like; cationic polyamino acids and their derivatives such as polylysine and their derivatives (Proceedings of Academy Sciences of the USA, 89, 6094 (1992)), polyethyleneimine, polyamineden-drimer and the like. "Hydrophobic polymers" are polymers which interact hydrophobically with genes, and include, for example, polyvinyl alcohol, polyvinyl pyrrolidone and the like. In addition, peptides such as AlkCWK18 may be employed.

Examples of the cationic liposomes include, but not limited to, LIPOFECTAMINE (trade name, Life Technologies, Inc., Rockville, MD, USA) containing DOSPA and DOPE (1:1), those containing DOTMA and DOPE (1:1), LIPOFECTACE (trade name, Life Technologies, Inc., Rockville, MD, USA) containing DDAB and DOPE (1:2.5), ELLFECTIN (trade name, Life Technologies, Inc., Rockville, MD, USA) containing TM-TPS and DOPE (1:1.5), and the like. Further, a mixture of said cationic lipid and cholesterol includes, for example, DMRIE-C (Life Technologies, Inc., Rockville, MD, USA) containing DMRIE and cholesterol (1:1 by mole).

Oligonucleotides may be contained in a membrane-fused liposome, or may be complexed with a liposome. Such membrane-fused liposomes includes, for example, HVJ-liposome.

Materials which promote transportation of a gene into nucleus include a mixture of HMG-1 and 2 (high mobility group-1, 2 mixture: Experimental Medicine, 12, 184 (1994)) and the like.

The ratio of collagen to oligonucleotide in the preparation for transferring an oligonucleotide of the present invention is important in that collagens attain: 1) protection of the oligonucleotide from a nuclease or other proteins, 2) sustained-release of the oligonucleotide, 3) local retention of the oligonucleotide to maintain locally a high concentration of the oligonucleotide, 4) promotion to transfer the oligonucleotide into cells. The ratio of collagen to oligonucleotide may be expressed as the number of a negative charge of the oligonucleotide in one molecule of collagen, as the chain length of the oligonucleotide may be appropriately selected depending on the particular object. Preferred number of a negative charge of the oligonucleotide in a molecule of collagen in the preparation for transferring an oligonucleotide of the present invention is 10 - 5,000, preferably 10 - 2,500, and more preferably 10 - 1000.

The preparation for transferring an oligonucleotide of the present invention may further comprise a pharmaceutically acceptable additive in addition to the oligonucleotide and the collagen.

For example, such additives include salts, amino acids and amines, which are used to adjust pH of the solution or the suspension type of the preparation for transferring an oligonucleotide; or those that control the release of oligonucleotide from a preparation for transferring an oligonucleotide in a form of a highly viscous solution or suspension, or a sponge, film, cylindrical and powdery. Examples of salts, amino acids and amines include tris(hydroxymethyl)aminomethane, sodium citrate, sodium dihydrogen citrate, trisodium phosphate, sodium hydrogen phosphate, anhydrous monosodium phosphate, anhydrous sodium dihydrogen phosphate, lysine, arginine and the like.

Additives that control the release include, for example, monosaccharide, disaccharide, trisaccharide or higher oligosaccharides or sugar alcohols or derivatives thereof; amino acids, organic acids having two or more carboxyl groups; gelatin, albumin, fibrin, alginic acid, agarose, gum arabic, and the like.

Monosaccharides include, for example, glucose, galactose, fructose and the like, and preferably glucose. Disaccharides include, for example, sucrose, maltose, lactose, trehalose and the like.

Sugar alcohols include, for example, sorbitol, mannitol, and preferably sorbitol.

Sugar derivatives include, for example, deoxy sugar, amino sugar, phosphate esters and disaccharides consisting of them.

Amino acids include, for example, pharmaceutically acceptable amino acids and salts thereof, and include acidic amino acids such as glutamic acid, aspartic acid; basic amino acids such as lysine, arginine, histidine; glycine, alanine, methionine, proline, cystine, serine, threonine, asparagine, glutamine, isoleucine, cysteine, tyrosine, tryptophane, leucine and the like. Amino acids as used herein further include amino acids that contains a basic or neutral side chain. Salts of amino acids include, for example, sodium salt, potassium salt.

Organic acids having two or more carboxy groups and salts thereof preferably include, for example, organic acids with two or three carboxy groups and salts thereof, and more preferably organic acids of saturated or unsaturated fatty series and salts thereof. Organic acids with two or three carboxy groups and salts thereof include, for example, citric acid, tartaric acid, succinic acid, malic acid, fumaric acid, and salts thereof. For example, citric acid, tartaric acid and salts thereof are preferred. Alternatively, an inactivated adenovirus having an ability to release the content of endosome, CHEMS (cholesterol hemisuccinate morpholine salt) or the like may be used in order to inhibit decomposition of oligonucleotide at endosome in cells.

The preparation for transferring an oligonucleotide of the present invention may include those containing any one of the sugars, the amino acids and the organic acids with two or more carboxyl groups as described above; and those containing two or three of any combination thereof.

Control of release using additives may be adjusted by changing the content of the additives. For example, the content of the additives should be decreased to extend the release period, whereas the content of the additives should be increased to shorten the period. The amount of additives may be selected from 0 - about 19,800 w/w% based on collagen. Preferred range is about 0 - about 1,890 w/w%, more preferably, about 0 - about 900 w/w%, and more preferably about 0 - 300 w/w%.

The preparation for transferring an oligonucleotide of the present invention may be in a form of solution or suspension, or in a form of a sponge, cylinder or powder formed from the solution or the suspension. The preparation for transferring an oligonucleotide of the present invention in a form of solution or suspension has a pH range from 4 to 8, and contains a collagen in the amount of 0.001 % to 10 %(w/w), preferably 0.1% to 5%(w/w). The preparation in a form of sponge, pellet or powder contains a collagne in the amount of 5 - 99 % (w/w), preferably 10 - 70 %(w/w), and more preferably 25 - 50 %(w/w).

The preparations of the present invention may be administered, for example, transdermally, subctaneously, intradermally, intramuscularly, intracerebrally, interstitially, intravascularly. They may be administered once every 3 days to once in about two months, preferably once a week to once a month.

The dose of the present preparation may be easily adjusted by changing the liquid volume in case of the preparations in a form of solution or suspension; the diameter and length in case of the cylindrical preparations; and the volume or weight in case of the a preparation in a powder form. Further, the dose may be also adjusted by changing the amount of oligonucleotides contained in the preparations.

Optimal dose of the preparations of the present invention may vary depending on the disease, site of application, method of administration, dosage form, sex, age, condition of the patient and the like. The amount of oligonucleotide contained in the preparation is 0.001 mg/kg to 40 mg/kg, preferably 0.01 mg/kg to 30 mg/kg of the subject.

The preparations for transferring an oligonucleotide of the present invention in a solution or suspension form may be prepared, for example:
1) a process wherein a desired oligonucleotide solution is added to a collagen solution, and an additive is dissolved as needed in the mixture to obtain a preparation in a homogeneous solution or a suspension form;
2) a process wherein a desired oligonucleotide solution is added to a collagen solution added with an additive as needed, and the mixture is mixed to obtain a preparation in a homogeneous solution or a suspension form;
3) a process wherein a desired oligonucleotide solution added with an additive as needed is added to a collagen solution, and the mixture is mixed to obtain a preparation in a homogeneous solution or a suspension form;
4) a process wherein a collagen solution added with an additive as needed is dried, and the resulting dried product is dissolved in a desired oligonucleotide solution to obtain a preparation in a homogeneous solution or a suspension form;
5) a process wherein a collagen solution is dried and the dried product is dissolved in a desired oligonucleotide solution added with an additive as needed, to obtain a preparation in a homogeneous solution or a suspension form;
6) a process wherein a desired oligonucleotide solution is dried and the dried product is dissolved in a collagen solution added with an additive as needed, to obtain a preparation in a homogeneous solution or a suspension form; and
7) a process wherein a desired oligonucleotide solution added with an additive as needed is dried, and the resulting dried product is dissolved in a collagen solution to obtain a preparation in a homogeneous solution or a suspension form.

In the above methods, the suspension or the solution may be obtained controlling on the concentration of the oligonucleotide solution or a collagen solution.

The processes for preparing the preparation for transferring an oligonucleotide in a film form include, for example:
1) a process wherein a desired oligonucleotide solution is added to a collagen solution, and an additive is dissolved as needed in the mixture to obtain a homogeneous solution or a suspension, which is then slowly dried to obtain a preparation in a film form;
2) a process wherein a desired oligonucleotide solution is added to a collagen solution added with an additive as needed, and the mixture is mixed to obtain a homogeneous solution or a suspension, which is then slowly dried to obtain a preparation in a film form;
3) a process wherein a desired oligonucleotide solution added with an additive as needed is added to a collagen solution, and the mixture is mixed to obtain a homogeneous solution or a suspension, which is then slowly dried to obtain a preparation in a film form;
4) a process wherein a collagen solution added with an additive as needed is dried, and the dried product is dissolved in a desired oligonucleotide solution to obtain a homogeneous solution or a suspension, which is then slowly dried to obtain a preparation in a film form;
5) a process wherein a collagen solution is dried, and the dried product is dissolved in a desired oligonucleotide solution added with an additive as needed, to obtain a homogeneous solution or a suspension, which is then slowly dried to obtain a preparation in a film form;
6) a process wherein a desired oligonucleotide solution is dried, and the dried product is dissolved in a collagen solution added with an additive as needed, to obtain a homogeneous solution or a suspension, which is then slowly dried to obtain a preparation in a film form;
7) a process wherein a desired oligonucleotide solution added with an additive as needed is dried, and the dried product is dissolved in a collagen solution to obtain a homogeneous solution or a suspension, which is then slowly dried to obtain a preparation in a film form.

The processes for preparing a preparation for transferring an oligonucleotide in a sponge form include, for example:
1) a process wherein a desired oligonucleotide solution is added to a collagen solution, and an additive is dissolved as needed in the mixture to obtain a homogeneous solution or a suspension, which is then freeze-dried to obtain a preparation in a sponge form;
2) a process wherein a desired oligonucleotide solution is added to a collagen solution added with an additive as needed, and the mixture is mixed to obtain a homogeneous solution or a suspension, which is then freeze-dried to obtain a preparation in a sponge form;
3) a process wherein a desired oligonucleotide solution added with an additive as needed is added to a collagen solution, and the mixture is mixed to obtain a homogeneous solution or a suspension, which is then freeze-dried to obtain a preparation in a sponge form;
4) a process wherein a collagen solution added with an additive as needed is dried, and the dried product is dissolved in a desired oligonucleotide solution, to obtain a homogeneous solution or a suspension, which is then freeze-dried to obtain a preparation in a sponge form;
5) a process wherein a collagen solution is dried, and the dried product is dissolved in a desired oligonucleotide solution added with an additive as needed, to obtain a homogeneous solution or a suspension, which is then freeze-dried to obtain a preparation in a sponge form.

The processes for preparing a preparation for transferring an oligonucleotide in a powder form include, for example:
1) a process wherein a desired oligonucleotide solution is added to a collagen solution, and an additive is dissolved as needed in the mixture to obtain a homogeneous solution, which is then spray-dried to obtain a preparation in a powder form;
2) a process wherein a desired oligonucleotide solution is added to a collagen solution added with an additive as needed, and the mixture is mixed to obtain a homogeneous solution, which is then spray-dried to obtain a preparation in a powder form;
3) a process wherein a desired oligonucleotide solution added with an additive as needed is added to a collagen solution, and the mixture is mixed to obtain a homogeneous solution, which is then spray-dried to obtain a preparation in a powder form;
4) a process wherein a collagen solution added with an additive as needed is dried, and the dried product is dissolved in a desired oligonucleotide solution to obtain a homogeneous solution, which is then spray-dried to obtain a preparation in a powder form;
5) a process wherein a collagen solution is dried, the dried product is dissolved in a desired oligonucleotide solution added with an additive as needed to obtain a homogeneous solution, which is then spray-dried to obtain a preparation in a powder form;
6) a process wherein a desired oligonucleotide solution is added to a collagen solution, and an additive is dissolved as needed in the mixture to obtain a homogeneous solution or a suspension, which is then freeze-dried, and the resulting sponge is ground to obtain a preparation in a powder form;
7) a process wherein a desired oligonucleotide solution is added to a collagen solution added with an additive as needed, and the combination is mixed to obtain a homogeneous solution or a suspension, which is then freeze-dried, and the resulting sponge is ground to obtain a preparation in a powder form;
8) a process wherein a desired oligonucleotide solution added with an additive as needed is added to a collagen solution, and the mixture is mixed to obtain a homogeneous solution or a suspension, which is then freeze-dried, and the resulting sponge is ground to obtain a preparation in a powder form;
9) a process wherein a collagen solution added with an additive as needed is dried, and the dried product is dissolved in a desired oligonucleotide solution to obtain a homogeneous solution or a suspension, which is then freeze-dried, and the resulting sponge is ground to obtain a preparation in a powder form;
10) a process wherein a collagen solution is dried, and the dried product is dissolved in a desired oligonucleotide solution added with an additive as needed, to obtain a homogeneous solution or a suspension, which is then freeze-dried, and the resulting sponge is ground to obtain a preparation in a powder form;
11) a process wherein a desired oligonucleotide solution is added to a collagen solution, into which are added an additive as needed, the resulting suspension is filtered, and the resulting precipitate is directly used or ground to obtain a preparation in a powder form;
12) a process wherein a desired oligonucleotide solution is added to a collagen solution added with an additive as needed, the resulting suspension is filtered, and the resulting precipitate is used directly or ground to obtain a preparation in a powder form;
13) a process wherein a desired oligonucleotide solution added with an additive as needed is added to a collagen solution, the resulting suspension is filtered and the resulting precipitate is used directly or ground to obtain a preparation in a powder form;
14) a process wherein a collagen solution added with an additive as needed is dried, the resulting dried product is dissolved in a desired oligonucleotide solution, the resulting suspension is filtered and the resulting precipitate is used directly or ground to obtain a preparation in a powder form;
15) a process wherein a collagen solution is dried, the dried product is dissolved in a desired oligonucleotide solution added with an additive as needed, the resulting suspension is filtered and the resulting precipitate is used directly or ground to obtain a preparation in a powder form;
16) a process wherein a desired oligonucleotide solution is dried, the dried product is dissolved in a collagen solution added with an additive as needed, the resulting suspension is filtered and the resulting precipitate is used directly or ground to obtain a preparation in a powder form;
17) a process wherein a desired oligonucleotide solution added with an additive as needed is dried, the dried product is dissolved in a collagen solution, the resulting suspension is filtered and the resulting precipitate is used directly or ground to obtain a preparation in a powder form.

The processes for preparing a preparation for transferring an oligonucleotide in a cylindrical form include, for example:
1) a process wherein a desired oligonucleotide solution is added to a collagen solution, into which are dissolved an additive as needed, the resulting homogeneous solution is spray-dried, and the resulting powder is compressed into cylinders;
2) a process wherein a desired oligonucleotide solution is added to a collagen solution added with an additive as needed, the resulting homogeneous solution is spray-dried, and the resulting powder is compressed into cylinders;
3) a process wherein a desired oligonucleotide solution added with an additive as needed is added to a collagen solution, the resulting homogeneous solution is spray-dried, and the resulting powder is compressed into cylinders;
4) a process wherein a collagen solution added with an additive as needed is dried, the dried product is dissolved in a desired oligonucleotide solution, the resulting homogeneous solution is spray-dried, and the resulting powder is compressed into cylinders;
5) a process wherein a collagen solution is dried, the dried product is dissolved in a desired oligonucleotide solution added with an additive as needed, the resulting homogeneous solution is spray-dried, and the resulting powder is compressed into cylinders;
6) a process wherein a desired oligonucleotide solution is added to a collagen solution, into which are dissolved an additive as needed, the resulting homogeneous solution or suspension is freeze-dried, the resulting sponge is ground to give powder which is compressed into cylinders;
7) a process wherein a desired oligonucleotide solution is added to a collagen solution added with an additive as needed, the resulting homogeneous solution or suspension is freeze-dried, and the resulting sponge is ground to give powder which is compressed into cylinders;
8) a process wherein a desired oligonucleotide solution added with an additive as needed is added to a collagen solution, the resulting homogeneous solution or suspension is freeze-dried, and the resulting sponge is ground to give powder which is compressed into cylinders;
9) a process wherein a collagen solution added with an additive as needed is dried, the dried product is dissolved in a desired oligonucleotide solution, the resulting homogeneous solution or suspension is freeze-dried, and thus obtained sponge is ground to give powder, which is compressed into cylinders;
10) a process wherein a collagen solution is dried, the dried product is dissolved in a desired oligonucleotide solution added with an additive as needed, the resulting homogeneous solution or suspension is freeze-dried, and thus obtained sponge is ground to give powder, which is compressed into cylinders;
11) a process wherein a desired oligonucleotide solution is added to a collagen solution, into which are dissolved an additive as needed, the resulting suspension is filtered, and thus obtained precipitate is , directly or after ground into powder, compressed into cylinders;
12) a process wherein a desired oligonucleotide solution is added to a collagen solution added with an additive as needed, the resulting suspension is filtered, and thus obtained precipitate is directly or after ground into powder, compressed into cylinders;
13) a process wherein a desired oligonucleotide solution added with an additive as needed is added to a collagen solution, the resulting suspension is filtered, and thus obtained precipitate is, directly or after ground into powder, compressed into cylinders;
14) a process wherein a collagen solution added with an additive as needed is dried, the resulting dried product is dissolved in a desired oligonucleotide solution, the resulting suspension is filtered, and thus obtained precipitate is, directly or after they are ground into powder, compressed into cylinders;
15) a process wherein a collagen solution is dried, the dried product is dissolved in a desired oligonucleotide solution added with an additive as needed, the resulting suspension is filtered, and thus obtained precipitate is, directly or after ground into powder, compressed into cylinders;
16) a process wherein a desired oligonucleotide solution is dried, the dried product is dissolved in a collagen solution added with an additive as needed, the resulting suspension is filtered, and thus obtained precipitate is, directly or after ground into powder, compressed into cylinders;
17) a process wherein a desired oligonucleotide solution added with an additive as needed is dried, the dried product is dissolved in a collagen solution, the resulting suspension is filtered, and thus obtained precipitate is, directly or after ground into powder, compressed into cylinders;
18) a process wherein a desired oligonucleotide solution is added to a collagen solution, into which are dissolved an additive as needed, the resulting homogeneous solution or suspension is freeze-dried to give sponge, which is compressed into cylinders;
19) a process wherein a desired oligonucleotide solution is added to a collagen solution added with an additive as needed, the resulting homogeneous solution or suspension is freeze-dried to give sponge, which is compressed into cylinders;
20) a process wherein a desired oligonucleotide solution added with additive as needed is added to a collagen solution, the resulting homogeneous solution or suspension is freeze-dried to give sponge, which is compressed into cylinders;
21) a process wherein a collagen solution added with an additive as needed is dried, the dried product is dissolved in a desired oligonucleotide solution, the resulting homogeneous solution or suspension is freeze-dried to give sponge, which is compressed into cylinders;
22) a process wherein a collagen solution is dried, the dried product is dissolved in a desired oligonucleotide solution added with additives, the resulting homogeneous solution or suspension is freeze-dried to give sponge, which is compressed into cylinders;
23) a process wherein a desired oligonucleotide solution is added to a collagen solution, into which are dissolved an additive as needed, the resulting homogeneous solution or suspension is freeze-dried to give sponge, which is, after addition of water or the like, kneaded, extruded into cylinder through a nozzle and dried;
24) a process wherein a desired oligonucleotide solution is added to a collagen solution added with an additive as needed, the resulting homogeneous solution or suspension is freeze-dried to give sponge, which is, after addition of water or the like, kneaded, extruded into cylinder through a nozzle and dried;
25) a process wherein a desired oligonucleotide solution added with an additive as needed is added to a collagen solution, the resulting homogeneous solution or suspension is freeze-dried to give sponge, which is, after addition of water or the like, kneaded, extruded into cylinder through a nozzle and dried;
26) a process wherein the collagens solution added with an additive as needed is dried, the dried product is dissolved in a desired oligonucleotide solution, the resulting homogeneous solution or suspension is freeze-dried to give sponge, which is, after addition of water or the like, kneaded, extruded into cylinder through a nozzle and dried;
27) a process wherein a collagen solution is dried, the dried product is dissolved in a desired oligonucleotide solution added with an additive as needed, the resulting homogeneous solution or suspension is freeze-dried to give sponge, which is, after addition of water or the like, kneaded, extruded into cylinder through a nozzle and dried;
28) a process wherein a desired oligonucleotide solution is added to a collagen solution, into which are dissolved an additive as needed, the resulting homogeneous solution is spray-dried to give powder, which is, after addition of water or the like, kneaded, extruded into cylinder through a nozzle and dried;
29) a process wherein a desired oligonucleotide solution is added to a collagen solution added with an additive as needed, the resulting homogeneous solution is spray-dried to give powder, which is, after addition of water or the like, kneaded, extruded into cylinder through a nozzle and dried;
30) a process wherein a desired oligonucleotide solution added with an additive as needed is added to a collagen solution, the resulting homogeneous solution is spray-dried to give powder, which is, after addition of water or the like, kneaded, extruded into cylinder through a nozzle and dried;
31) a process wherein a collagen solution added with an additive as needed is dried, a desired oligonucleotide solution is added to the dried product, then kneaded, extruded into cylinder through a nozzle and dried;
32) a process wherein a collagen solution is dried, the dried product is added to a desired oligonucleotide solution added with an additive as needed, then kneaded, extruded into cylinder through a nozzle and dried.

### Examples

The present invention is further illustrated by the following Examples and Experiments, but is not restricted by these Examples and Experiments in any way.

### Example 1

The preparation in a solution form for transferring an oligonucleotide which comprises atelocollagen at the final concentration of 0.5 %, was prepared by mixing the phosphorothioate antisense oligonucleotide (5'-CTCGTAGGCGTTGTAGTTGT-3' (SEQ ID NO:1); molecular weight, about 6,500) (manufactured by Sawaday) having a sequence complementary to a sequence from 4196 bp to 4216 bp of the fibroblast growth factor HST-1 (FGF4) gene (described in Proc. Natl. Acad. Sci. USA, 84, 2890-2984 (1987)) with a neutral solution of atelocollagen (atelocollagen implant manufactured by KOKEN CO., LTD.; 2% atelocollagen solution) to be the final concentration to 10 µmol/L.

### Example 2

The preparation in a solution form for transferring an oligonucleotide was prepared by mixing the phosphorothioate type antisense oligonucleotide (AS-ODC, 5'-TCATGATTTCTTGATGTTCC-3' (SEQ ID NO: 2) manufactured by ESPEC OLICO SERVICE CORP.) having a sequence complementary to a sequence from 319 bp to 338 bp in the ornithine decarboxylase gene with a neutral solution of atelocollagen (atelocollagen implant manufactured by KOKEN CO., LTD.; 3.5 w/w% atelocollagen solution; the final concentration, 1.75 w/w%) to bring the final concentration of the oligonucleotide to 0.5, 1.5, 2, 2.5 mmol/L.

### Example 3

The preparation for transferring an oligonucleotide which contains a liposome was prepared by mixing AS-ODC with 0.5 µL of a neutral solution of atelocollagen (atelocollagen implant manufactured by KOKEN CO., LTD.; 3.5 w/w% atelocollagen solution; final concentration, 1.75 wt%) and 1.5 µL of Transfast (Promega) to bring the final concentration of the oligonucleotide to 1.0 mmol/L.

### Example 4

The preparation in a solution form for transferring an oligonucleotide as prepared in Example 1 (3 mL) was poured into a dish made of polystyrene (diameter; 35mm), and the dish was stand still in a desiccator containing silica gel at 5 °C to be slowly dried while keeping on an even keel, to give a preparation in a film form for transferring an oligonucleotide containing 32.5 µg of the oligonucleotide per 1 mg of the collagen.

### Example 5

The preparation in a solution form for transferring an oligonucleotide as prepared in Example 1 (3 mL) was poured into a dish made of polystyrene (diameter; 35mm), and the dish was frozen at - 40 °C, then dried overnight under reduced pressure at room temperature, to give a preparation in a sponge form for transferring an oligonucleotide containing 32.5 µg of the oligonucleotide per 1 mg of the collagen.

### Example 6

After adding water (52.5 g) and a 10 mg/mL glucose solution (10 mL) to a 2 w/w% atelocollagen solution (17.5 g), the mixture was added with a 5 mg/mL solution (10 mL) of the phosphorothioate type antisense oligonucleotide (5'-CTCGTAGGCGTTGTAGTTGT-3' (SEQ ID NO:1); molecular weight: about 6.500), and the mixture was mixed. The resulting solution was frozen at - 40 °C, and then dried overnight under reduced pressure at room temperature. A proper amount of distilled water was added to the freeze-dried product thus obtained and the mixture was kneaded. Then, the kneaded product was extruded through a nozzle into rods, and dried to give preparations in a rod form. This gave gene preparations in rod forms containing 1 mg of the oligonucleotide per 10 mg of the preparation.

### Example 7

Fifty µl of a physiological saline containing 10 mg/ml of the phosphorothioate type antisense oligonucleotide (AS-ODC, 5'-TCATGATTTCTTGATGTTCC-3') (SEQ ID NO: 2) (manufactured by ESPEC OLIGO SERVICE CORP.) having a sequence complementary to a sequence from 319 b to 338 b of the ornithine decarboxylase gene was mixed with 50 µl of an atelocollagen solution (3.5 %) at room temperature, to give a preparation in a solution form for transferring an oligonucleotide containing 5 g/µl of AS-ODC and 1.75 % of atelocollagen.

### Reference Example 1

A solution of the phosphorothioate type antisense oligonucleotide was prepared by the same manner of Example 1, except that the neutral solution of atelocollagen was replaced with distilled water.

### Reference Example 2

A preparation in a solution form was obtained by the same manner of Example 1, except that the phosphorothioate type antisense oligonucleotide having a sequence complementary to a sequence from 4196 b to 4216 b of the fibroblast growth factor HST-1 (FGF 4) gene (described in Proc. Natl. Acad. Sci. USA, 84, 2890 - 2984 (1987)) was replaced by the phosphorothioate type sense oligonucleotide (5'-ACAACTACAACGCCTACGAG-3')(SEQ ID NO: 3) having the same sequence.

### Reference Example 3

The phosphorothioate type antisense oligonucleotide (5'-CTCGTAGGCGTTGTAGTTGT-3')(SEQ ID NO: 1); molecular weight about 6,500) having a sequence complementary to a sequence from 4196 b to 4216 b of the fibroblast growth factor HST-1 (FGF 4) gene (described in Proc. Natl. Acad. Sci. USA, 84, 2890- 2984 (1987)) was dissolved in a - phosphate buffer (500 µL) to bring the concentration to 20 µmol/L. The solution was mixed with a phosphate buffer (500 µL) containing lipofectamine (GIBCO BRL) (25 µ/L) dissolved therein, and then the mixture was stood still to react at room temperature for 20 minutes to obtain a oligonucleotide-liposome preparation (final concentration of the oligonucleotide, 10 µmol/L).

### Reference Example 4

According to the procedures described in Example 2, except that the phosphate buffer was added instead of As-ODC, an atelocollagen solution containing 1.75 w/w% atelocollagen was prepared.

### Reference Example 5

According to the procedures described in Example 2, except that the neutral solution of atelocollagen was replaced by a phosphate buffer, a solution of the phosphorothioate type antisense oligonucleotide containing AS-ODC at the concentration of 1.0 mmol/L was prepared.

### Reference Example 6

According to the procedures described in Example 2, except that the phosphorothioate type scramble oligonucleotide (5'-AGTACTAAAGAACTACAAGG-3')(SEQ ID NO:4)(ESPEC OLIGO SERVICE CORP.) containing a heterologous sequence was contained at the concentration of 1.0 mmol/L, instead of AS-ODC, a phosphorothioate type scramble oligonucleotide composition in a solution form was obtained.

### Reference Example 7

The liposome preparation was obtained by mixing a phosphate buffer and a phosphate buffer containing transfast (Promega) dissolved therein (15 µL) to bring the final AS-ODC concentration to 1.0 mmol/L.

### Experiment 1

The preparation for transferring an oligonucleotide as prepared in Example 1 (2 µL) was mixed with a PBS (-) solution (100 µL) containing 20 % fetal bovine serum, and the mixture was stood still at 37 °C. After mixing, the aliquots of the mixed solution were took after 30, 60, 180 minutes, and subjected to 3% agarose gel electrophoresis, to evaluate the primary structure of the phosphorothioate type antisense oligonucleotide in the preparation. The results are shown in Figure 1. For the phosphorothioate type antisense oligonucleotide in the oligonucleotide-type preparation as prepared in Example 1, a band was observed at the migrated position of untreated phosphorothioate type antisense oligonucleotide even 180 minutes after mixed with PBS (-) solution containing 20 % fetal bovine serum. On the other hand, the preparation of Reference Example 1 exhibited no band after 30 minutes. The results showed that the phosphorothioate type oligonucleotide in the preparation of the present invention was protected from degradation with an enzyme.

### Experiment 2

Human testicular embryonal carcinoma cells (NEC-8) (10 x 10⁵) producing HST-1 were transplanted into the testes of the 10-weeks old male nude mice. Ten days after the transplantation, the preparation as prepared in Example 1 was administered at the dose of 50 µl per testis. 10, 20, and 30 days after the administration, the nude mice were sacrificed, and the tumor cells in the testes were weighed. The results are shown in Figure 2. The growth of the testicular embryonal carcinoma cells in the testes of the nude mice was most strongly inhibited by the preparation of Example 1, compared to the gel composition of Reference Example 2 and the liposome composition of Reference Example 3 (each administered at 50 µl/testis). Particularly, the liposome composition of Reference Example 3 showed an inhibition effect similar to that of the preparation for transferring an oligonucleotide of Example 1 until 20 days, but did not show any growth inhibition effect after 30 days. This result shows that the preparation of the present invention retains in the testes of the nude mice after the administration, protects the phosphorothioate type oligonucleotide which inhibits HST-1 production in the nude mice from enzymatic degradation, and further has an activity to inhibit the growth of tumor cells effectively for a long period by locally and gradually releasing this phosphorothioate type oligonucleotide.

### Experiment 3

5 x 10⁵ of the human stomach cancer cells (MKN 45 cell) (Japan Health Sciences Foundation) cultured in DMEM (500 µL) containing 10 % fetal bovine serum were added with 5 µL of the preparation in a solution form for transferring an oligonucleotide as prepared in Example 2 (containing 1.0 mmol/L AS-ODC) (final concentration, 10 µmol/L), and cultured at 37 °C. Twenty four hours after the addition, the medium containing the preparation for transferring an oligonucleotide was replaced with a fresh medium, and then cultivation was conducted for 6 days while replacing medium every 24 hours. After the addition, the number of cells were measured every 2 days using 3-[4,5-dimethylthiazol-2-yl]-2,5-diphenyl-2H-tetrazolium bromide (MTT assay). Similarly, the atelocollagen solution as prepared in Reference Example 4, the phosphorothioate type antisense oligonucleotide solution as prepared in Reference Example 5, and the phosphorothioate type scramble composition for transferring an oligonucleotide in a solution form as prepared in Reference Example 6 were added to MKN 45 cells, and the number of cells were measured. The results are shown in Figure 3. When the preparation in a solution form of Example 2 was administered, the growth of MKN 45 cells was significantly inhibited compared to the control with nothing added. Further, MKN 45 cells were decreased in number after 6 days, compared to the number at the time of addition. On the other hand, when the preparations of Reference Example 4 (atelocollagen solution), Reference Example 5 (containing AS-ODC alone), and Reference Example 6 (containing the phosphorothioate type scramble oligonucleotide and atelocollagen) were added, the growth of MKN 45 cells were inhibited compared to control, but the effect was lower than that of Example 2. These results show that the growth inhibition - effect of the phosphorothioate type antisense oligonucleotide on tumor cells may be enhanced by mixing said preparation with atelocollagen to prepare the preparation of the present invention, and that the mechanism to inhibit the growth of tumor cells by the preparation of the present invention is derived not only from coexistence of atelocollagen or the phosphorothioate type oligonucleotide with the target cells, but from the growth inhibition and cytotoxicity of the target cell by the phosphorothioate type antisense oligonucleotide.

### Experiment 4

The preparations in a solution form for transferring an oligonucleotide as prepared in Example 2 (containing 0.5, 1, 1.5, 2, 2.5 mmol/L of AS-ODC) were added to 5 x 10⁵ of human rhabdomyosarcoma cells (RD cells) (Japan Health Sciences Foundation) which had been cultured in 500 µL of DMEM containing 10% fetal bovine serum (the final concentration, 5, 10, 15, 20, 25 µmol/L), and the cells were cultured at 37 °C. Twenty four hours after the addition, the media containing the preparations were replaced with a fresh medium, then cultivation was conducted for 5 days while replacing the media every 24 hours. One and 35 days after addition, survival rates of the cells were measured using trypan-blue staining. The results are shown in Figure 4. All of the preparations measured exhibited the decrease in survival ratio of RD cells. Even at the lowest concentration, i.e., 5 µmol/L, the preparation exhibited the cytotoxicity effect.

### Experiment 5

- The liposome-containing preparation for transferring an oligonucleotide as prepared in Example 3 (5 µL) was added to 5 x 10⁵ of human rhabdomyosarcoma cells (RD cells) which had been cultured in 500 µL of DMEM containing 10% fetal bovine serum, and the cells were cultured at 37 °C. Twenty four hours after the addition, the medium containing the preparation was replaced with a fresh medium, then cultivation was conducted for 8 days while replacing medium every 24 hours. 2, 4, 6 and 8 days after the addition, survival ratio of the cells was measured using trypan-blue staining. 2, 4 and 6 days after the addition, the ornithine decarboxylase activity in RD cells was also measured. Similarly, the liposome preparation of Reference Example 7 was added, and then the survival of RD cells and the ornithine decarboxylase activity in the cells were investigated. The results are shown in Figures 5 and 6. Both the liposome-containing preparation for transferring an oligonucleotide of Example 3 and the liposome preparation of Reference Example 7 decreased the survival of RD cells, although the preparation for transferring an oligonucleotide exhibited the stronger cytotoxicity. Similarly, both liposome-containing preparation for transferring an oligonucleotide and the liposome preparation decreased the ornithine decarboxylase activity in RD cells, with the degree of decrease by the preparation for transferring an oligonucleotide being stronger than that by the liposome preparation. This is because the preparation for transferring an oligonucleotide containing liposome of Example 3 inhibited the expression of the target gene, ornithine decarboxylase gene, more strongly compared to the liposome preparation of Reference Example 7, and decreased the RD cell number. These results show that the preparation for transferring an oligonucleotide of the present invention, even when containing a liposome, enhances cytotoxicity by the phosphorothioate type oligonucleotide, and is superior in such enhancement to the conventional liposome preparations.

### Experiment 6

5 x 10⁷ RD cells over-expressing ornithine decarboxylase were transplanted subcutaneously on the back of the 4-week old male nude mice. Seven days after the transplantation, the preparation for transferring an oligonucleotide as prepared in Example 7 was administered in the site of the transplantation, in the back muscle opposed to the transplantation, and in the peritoneal cavity. Similarly, the atelocollagen solution of Reference Example 4, the liposome preparation of Reference Example 7, and the phosphate buffer (Control) were respectively administered to the site where the RD cells were transplanted. After the administration, the nude mice were sacrificed every 7 days, and the RD cells were weighed. The results are shown in Figures 7, 8 and 9. Figure 7 shows the comparison between the preparations of Example 7 and Reference Example 4 administered to the site where the RD cells were transplanted. Figure 8 shows the comparison between the preparations of Example 7 and Reference Example 7 administered at the site where the RD cells were transplanted. Figure 9 shows the result of administration of the preparation of Example 7 to the site where the RD cells were transplanted (local injection), intramuscularly (intramuscular injection) - and intraperitoneally (intraperitoneal injection). The growth of RD cells in the back muscle of the nude mice was most strongly inhibited by the preparation for transferring an oligonucleotide as prepared in Example 7 in all nude mice when administered at the RD transplantation site, intramuscularly at the opposite side of the transplantation, and intraperitoneally (Fig. 9), compared to the atelocollagen solution of Reference Example 4 (Fig. 7), the liposome preparation of Reference Example 7 (Fig. 8) and the phosphate buffer. Particularly, the liposome preparation of Reference Example 7 exhibited an inhibition corresponding to that of the preparation for transferring an oligonucleotide of Example 7 until 7 days, but exhibited no inhibition of the growth after 14 days. These facts show that the preparation for transferring an oligonucleotide of the present invention inhibits the growth of RD cells more strongly compared to the liposome preparation, that such inhibition of growth of RD cells may last for a long time, and that such inhibition of growth may not be affected by the site of administration. This result suggests that the oligonucleotide preparation of the present invention has an efficient inhibition effect on the growth of tumor cells, which lasts for a long time by protection Figures 10 and 11 show the cumulative survival curve of RD cell-transplanted nude mice the phosphorothioate type antisense oligonucleotide from degradation by an enzyme in the nude mice after administration, and gradually releasing the phosphorothioate type oligonucleotide. Figure 10 shows the comparison between preparations of Example 7 and Reference Example 4 when administered at the site of RD-transplantation. Figure 11 - shows the comparison between the preparations of Example 7 and Reference Example 7 when administered at the site of RD transplantation. When the preparation for transferring an oligonucleotide of Example 7 was administered at the site of RD-transplantation, the average viable period of the mice was 52.6 days, which was greatly elongated compared to the nude mice administered with the phosphate buffer (average, 20.5 days), the atelocollagen solution of Reference Example 4 (average 40.3 days) (Fig. 10) and the liposome preparation of Reference Example 7 (average 37.8 days) (Fig. 11). This result shows that the inhibition of growth of RD cells which can be obtained by administration of the present preparation for transferring an oligonucleotide will enhance the survival of the nude mice that had been transplanted with RD cells. Further, ornithine decarboxylase activities in RD cells after administration of the preparation for transferring an oligonucleotide of Example 7, the atelocollagen solution of Reference Example 4, the liposome preparation of Reference Example 7 and the phosphate buffer are shown in Figures 12 and 13. Figure 12 shows the comparison between preparations of Example 7 and Reference Example 4 administered at the site of RD-transplantation. Figure 13 shows the comparison between preparations of Example 7 and Reference Example 7 when administered to the site of RD-transplantation. Ornithine decarboxylase activity in RD cells treated with the preparation for transferring an oligonucleotide is lower than those treated with the atelocollagen solution, the liposome preparation and the phosphate buffer. This suggest that ornithine decarboxylase production is - inhibited by administration of the preparation for transferring an oligonucleotide, and the inhibition of growth of RD cells observed upon administration of the preparation for transferring an oligonucleotide is obtained by inhibiting the expression of ornithine decarboxylase gene by the phosphorothioate type oligonucleotide contained in the preparation for transferring an oligonucleotide.

### Experiment 7

5 x 10⁷ MKN 45 cells over-producing ornithine decarboxylase were transplanted subctaneously to the back of 4-week old male nude mice. Seven days after transplantation, the preparation for transferring an oligonucleotide as prepared in Example 7 was administered at the site where MKN45 cells were transplanted. Similarly, the atelocollagen solution of Reference Example 4, the phosphorothioate type scramble oligonucleotide composition in a solution form of Reference Example 6 and the phosphate buffer (Control) were respectively administered at the site where MKN 45 cell had been transplanted. After administration, the nude mice were sacrificed every 7 days and MNK 45 cells were weighed. The result is shown in Figure 14. Growth of MKN 45 cells in the back muscle of the nude mice was most strongly inhibited by the preparation for transferring an oligonucleotide as prepared in Example 7 compared to the atelocollagen solution of Reference Example 4, the phosphorothioate scramble oligonucleotide composition in a solution form of Reference Example 6 and the phosphate buffer. This result suggests that the preparation for transferring an oligonucleotide of the - present invention inhibits the growth of MKN 45 cells, and hold the effect to inhibit the growth of MKN 45 cells for a long time. Figure 15 shows a cumulative survival curve of the nude mice transplanted with MKN cells. Average viable period of the nude mice administered with the preparation for transferring an oligonucleotide of Example 7 at the site of the transplantation was 48.5 days, which was greatly elongated compared to those administered with the atelocollagen solution of Reference Example 4 (39.1 days), the phosphorothioate scramble oligonucleotide composition in a solution form of Reference Example 6 (39.1 days), and the phosphate buffer (21.3 days). The result suggests that the inhibition of growth of MKN cells obtained by administration of the present oligonucleotide preparation will enhance the survival of the nude mice transplanted with MKN cells. Further, ornithine decarboxylase activities in MKN 45 cells on the 35^{th} days after administration of the preparation for transferring an oligonucleotide of Example 7, the atelocollagen solution of Reference Example 4, the phosphorothioate type scramble oligonucleotide composition in a solution form of Reference Example 6 and the phosphate buffer are shown in Figure 16. Ornithine decarboxylase activity in MKN 45 cells treated with the preparation for transferring an oligonucleotide was significantly lower than those treated with the atelocollagen solution, the phosphorothioate scramble oligonucleotide composition in a solution form and the phosphate buffer. This suggest that ornithine decarboxylase production is inhibited by administration of the preparation for transferring an oligonucleotide, and that such inhibition of the growth of MKN 45 cells observed upon administration - of the preparation for transferring an oligonucleotide is caused by the inhibition of expression of the ornithine decarboxylase gene by phosphorothioate type oligonucleotide contained in the oligonucleotide-transferring preparation.

### Experiment 8

5 x 10⁷ human large intestinal cancer cells (COLO201 cells) (Japan Health Sciences Foundation) over-producing ornithine decarboxylase were transplanted subctaneously to the 4-week old male nude mice. Seven days after the transplantation, the preparation for transferring an oligonucleotide as prepared in Example 7 was administered at the site where COLO201 had been transplanted. Similarly, the atelocollagen solution as prepared in Reference Example 4 and the phosphate buffer (Control) were respectively administered to the site where COLO201 cells had been transplanted. After the administration, the nude mice were sacrificed every 7 days, and COLO201 cells were weighed. The results are shown in Figure 17. Growth of COLO201 cells in the back muscle of the nude mice was most strongly inhibited by the preparation for transferring an oligonucleotide of Example 7, compared to the atelocollagen solution of Reference Example 4 and the phosphate buffer. The weight of COLO201 cells was also decreased compared to those at the time of transplantation. These results suggest that the present preparation for transferring an oligonucleotide inhibits the growth of COLO201 and kills COLO201 cells, and that such inhibition effect of growth of COLO201 cells may last for a long time. Figure 18 shows a cumulative - curve of nude mice transplanted with COLO201 cells. Average viable period of the nude mice treated with the preparation for transferring an oligonucleotide of Example 7 was 39.8 days, which was longer than those of the atelocollagen solution and the phosphate buffer (39.8 days and 20.6 days, respectively). This result suggests that such inhibition of growth of COLO201 obtained by administration of the present preparation for transferring an oligonucleotide enhanced the survival of the nude mice transplanted with COLO201. Further, Figure 19 shows the ornithine decarboxylase activity in COLO201 cells after 35 days of administration of the preparation for transferring an oligonucleotide of Example 7, the atelocollagen solution of Reference Example 4 and the phosphate buffer. Ornithine decarboxylase activity in COLO 201 cells treated with the preparation for transferring an oligonucleotide was lower than those treated with the atelocollagen solution and the phosphate buffer. This result suggests that administration of the preparation for transferring an oligonucleotide inhibits the production of ornithine decarboxylase. The result suggests that inhibition of growth of COLO 201 cells observed upon administration of the preparation for transferring an oligonucleotide is caused by inhibition of expression of ornithine decarboxylase gene by the phosphorothioate type oligonucleotide contained in the oligonucleotide transferring preparation.

### Experiment 9

The preparation in a solution form for transferring an oligonucleotide (containing 100 µmol/L AS-ODC and 0.175 % AS-- ODC)(10 µL) was added to 1 x 10⁵ mouse leiomyoma cells (LMFS cells) cultured in 0.5 mL of DMEM medium containing 10% fetal bovine serum, and the cells were cultured at 37 °C. Twenty four hours after the addition, the medium containing oligonucleotide preparation was replaced with a fresh medium, and then cultivation was conducted for 6 days while replacing a medium every 24 hours. After the addition, the number of cells and the ornithine decarboxylase activity were measured. The results are shown in Figures 20 and 21, respectively. After the addition of the preparation in a solution form for transferring an oligonucleotide of Example 7, the growth of LMFS cells was significantly inhibited compared to controls without addition, and the ornithine decarboxylase activity was reduced. The result suggests that the present preparation for transferring an oligonucleotide can inhibit the growth of LMFS, which exhibits extremely strong proliferative and metastatic properties, by recognizing the target base sequence specifically.

### INDUSTRIAL APPLICABILITY

The preparation comprising an oligonucleotide having a base sequence complementary to that of a target m-RNA, and a collagen (as well as a pharmaceutically acceptable additive) effectively inhibits the expression of the target m-RNA without inducing any side effect *in vivo*, and maintains the inhibition effect for a long period of time, whereby attaining gene therapy.

## Claims

1. A preparation for transferring a desired oligonucleotide into a target cell, which comprises a soluble type 1 collagen derived from dermis or a soluble type 1 collagen prepared by genetic engineering as an essential component, said preparation releasing a 5 to 30 mer oligonucleotide.

2. A preparation according to Claim 1, wherein the target cell is an animal cell.

3. A preparation according to Claim 2, wherein the target cell is an organ or a tissue which require to be treated for a subject disease, or its cell around.

4. A preparation according to any one of Claims 1 to 3, wherein the collagen is atelocollagen.

5. A preparation according to any one of Claims 1 to 4, wherein the molecular weight of the collagen is from about 300,000 to about 900, 000.

6. A preparation according to any one of Claims 1 to 5, wherein the preparation is a sustained-release formulation.

7. A preparation according to Claim 6, wherein the oligonucleotide is a DNA or a DNA derivative.

8. A preparation according to Claim 6, wherein the oligonucleotide is an RNA or an RNA derivative.

9. A preparation according to Claim 1, wherein the oligonucleotide has at least one phosphorothioate bond.

10. A preparation according to Claim 1, wherein the oligonucleotide contains as at least portion a base sequence that binds complementarily under physiological conditions to a sense or antisense strand of a gene encoding a protein that exhibits a physiological effect to disrupt the homeostasis of a living body.

## Patentansprüche

1. Zubereitung für das Transferieren eines gewünschten Oligonukleotids in eine Zielzelle, wobei die Zubereitung als wesentlichen Bestandteil ein von der Dermis herrührendes lösliches Typ 1-Kollagen oder ein lösliches Typ 1-Kollagen umfasst, das mittels Gentechnik hergestellt wurde, wobei die Zubereitung ein 5-bis 30-Mer-Oligonukleotid freisetzt.

2. Zubereitung nach Anspruch 1, wobei die Zielzelle eine Tierzelle ist.

3. Zubereitung nach Anspruch 2, wobei die Zielzelle ein Organ oder ein Gewebe ist, das der Behandlung im Hinblick auf eine gegebene Krankheit bedarf, oder die umgebende Zelle.

4. Zubereitung nach einem der Ansprüche 1 bis 3, wobei das Kollagen Atelokollagen ist.

5. Zubereitung nach einem der Ansprüche 1 bis 4, wobei das Molekulargewicht des Kollagens von etwa 300.000 bis etwa 900.000 ist.

6. Zubereitung nach einem der Ansprüche 1 bis 5, wobei die Zubereitung eine Formulierung für verzögerte Freisetzung ist.

7. Zubereitung nach Anspruch 6, wobei das Oligonukleotid eine DNA oder ein DNA-Derivat ist.

8. Zubereitung nach Anspruch 6, wobei das Oligonukleotid eine RNA oder ein RNA-Derivat ist.

9. Zubereitung nach Anspruch 1, wobei das Oligonukleotid mindestens eine Phosphorothioat-Bindung hat.

10. Zubereitung nach Anspruch 1, wobei das Oligonukleotid zumindest als Teil eine Basensequenz enthält, die unter physiologischen Bedingungen komplementär an einen Sense- oder Antisensestrang eines Gens bindet, das ein Protein kodiert, das einen physiologischen Effekt des Störens der Homöostase eines lebenden Körpers zeigt.

## Revendications

1. Préparation pour transférer un oligonucléotide désiré dans une cellule cible, qui comprend du collagène soluble de type 1 dérivé du derme ou du collagène soluble de type 1 préparé par génie génétique comme un composant essentiel, ladite préparation libérant un oligonucléotide de 5 à 30 mer.

2. Préparation selon la revendication 1, dans laquelle la cellule cible est une cellule animale.

3. Préparation selon la revendication 2, dans laquelle la cellule cible est un organe ou un tissu qui a besoin d'être traité chez un sujet atteint d'une maladie, ou sa cellule environnante.

4. Préparation selon l'une quelconque des revendications 1 à 3, dans laquelle le collagène est atélocollagène.

5. Préparation selon l'une quelconque des revendications 1 à 4, dans laquelle le poids moléculaire du collagène est d'environ 300 000 à environ 900 000.

6. Préparation selon l'une quelconque des revendications 1 à 5, dans laquelle la préparation est une formulation à libération prolongée.

7. Préparation selon la revendication 6, dans laquelle l'oligonucléotide est un ADN ou un dérivé d'ADN.

8. Préparation selon la revendication 6, dans laquelle l'oligonucléotide est un ARN ou un dérivé d'ARN.

9. Préparation selon la revendication 1, dans laquelle l'oligonucléotide a au moins une liaison phosphorothioate.

10. Préparation selon la revendication 1, dans laquelle l'oligonucléotide contient, au moins dans une portion, une séquence de bases qui se lie de manière complémentaire dans des conditions physiologiques à un brin sens ou antisens d'un gène codant une protéine qui présente un effet physiologique pour rompre l'homéostasie d'un corps vivant.


### SEQUENCE LISTING

<110> SUMITOMO PHARMACEUTICALS COMPANY LIMITED; KOKEN CO., LTD.
<120> OLIGONUCLEOTIDES-TRANSFERRING PREPARATIONS
<130> 663591HT
<150> JP 2000-184502
   <151> 2000-06-20
<160> 4
<210> 1
   <211> 20
   <212> DNA
   <213> Artificial Sequence
<220>
   <223> Phosphorothioate type anti-sense oligonucleotide
<400> 1
   ctcgtaggcg ttgtagttgt 20
<210> 2
   <211> 20
   <212> DNA
   <213> Artificial Sequence
<220>
   <223> Phosphorothioate type anti-sense oligonucleotide
<400> 2
   tcatgatttc ttgatgttcc 20
<210> 3
   <211> 20
   <212> DNA
   <213> Artificial Sequence
<220>
   <223> Phosphorothioate type sense oligonucleotide
<400> 3
   acaactacaa cgcctacgag 20
<210> 4
   <211> 20
   <212> DNA
   <213> Artificial Sequence
<220>
   <223> Phosphorothioate type scramble oligonucleotide
<400> 4
   agtactaaag aactacaagg 20